(19) **Europäisches Patentamt / European Patent Office / Office européen des brevets**

(11) **EP 3 821 712 B2**

(12) # NEW EUROPEAN PATENT SPECIFICATION
After opposition procedure

(45) Date of publication and mention
of the opposition decision:
**01.07.2026  Bulletin 2026/27**

(45) Mention of the grant of the patent:
**09.11.2022  Bulletin 2022/45**

(21) Application number: **20206614.8**

(22) Date of filing: **12.01.2018**

(51) International Patent Classification (IPC):
**A23C 9/12** (2006.01)  **A23C 9/123** (2006.01)
C12R 1/225 (2006.01)  C12R 1/46 (2006.01)
C12N 9/38 (2006.01)

(52) Cooperative Patent Classification (CPC):
(C-Sets available)
**A23C 9/1206; A23C 9/1238; C12N 1/205;
C12N 9/2402;** A23V 2200/00; A23V 2400/137;
A23V 2400/249; C12R 2001/225          (Cont.)

(54) **FERMENTED MILK PRODUCT OBTAINED BY AN IMPROVED PROCESS**

FERMENTIERTES MILCHPRODUKT ERHALTEN DURCH EINEM VERBESSERTEN VERFAHREN

PRODUIT LAITER FERMENTÉ OBTENU PAR UN PROCEDE AMELIORE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **13.01.2017  EP 17151378**

(43) Date of publication of application:
**19.05.2021  Bulletin 2021/20**

(62) Document number(s) of the earlier application(s) in
accordance with Art. 76 EPC:
**18704863.2 / 3 568 024**

(73) Proprietor: **Kerry Group Services International
Ltd
Tralee, Co. Kerry, V92 EH11 (IE)**

(72) Inventors:
• **BA, Zhaoyong
2970 Hoersholm (DK)**

• **BUCHHORN, Gaelle Lettier
2970 Hoersholm (DK)**
• **BULDO, Patrizia
2970 Hoersholm (DK)**
• **HOEGHOLM, Tina
2970 Hoersholm (DK)**
• **RUNGE, Mette Oehrstroem
2970 Hoersholm (DK)**
• **SCHOELER, Jeppe
2970 Hoersholm (DK)**
• **VOJINOVIC, Vojislav
2970 Hoersholm (DK)**

(74) Representative: **Bird & Bird Società tra Avvocati
S.r.l.
Via Porlezza, 12
20123 Milano (IT)**

(56) References cited:
WO-A1-2007/021204     WO-A1-2009/071539
WO-A1-2015/193449     WO-A1-2015/193459
US-A1- 2009 088 391    US-A1- 2015 086 675

(52) Cooperative Patent Classification (CPC): (Cont.)

C-Sets
A23V 2200/00, A23V 2200/3204, A23V 2400/137,
A23V 2400/249

**Description**

FIELD OF THE INVENTION

The present invention relates to a fermented milk product

BACKGROUND OF THE INVENTION

WO 2009/071539 discloses a lactase originating from *Bifidobacterium bifidum,* which is capable of very efficient hydrolysis in milk, and which is active over a broad pH range, including low pH, e.g. a pH below 5. The lactase may be used in processes for producing milk and fermented milk products, such as cheese, yogurt, butter, butter milk, sour cream etc., for reducing the content of lactose.

WO 2013/160413 discloses a method of producing a fermented milk product using a combination of glucose-negative lactic acid bacteria strains and a conventional lactase with an object of reducing the content of lactose in the fermented milk product while increasing the content of glucose.

EP-A1-2 957 180 in one embodiment discloses a method of producing a fermented milk product using a combination of a starter cultures and a conventional lactase with an object of reducing content of lactose and the level of post-acidification in the fermented milk product. EP-A1-2 957 180 in a second embodiment discloses a method of producing a fermented milk product using lactose-deficient lactic acid bacteria.

SUMMARY OF THE INVENTION

The object of the present invention is defined by the claims and relates to fermented milk products produced by an improved process as disclosed herein.

The object of the present invention is a fermented milk product comprising the starter culture of step 3) and the low pH stable lactase added in step 4), which is obtained by a process comprising the steps of

1) adding a starter culture comprising both at least one lactose-deficient *Streptococcus thermophilus* and at least one lactose-deficient *Lactobacillus delbrueckii* subsp. *bulgaricus* to a milk base,

2) fermenting the milk for a period of time until a target pH is reached, wherein the residual level of lactose at the end of fermentation is less than 25 mg/ml, less than 20 mg/ml, less than 15 mg/ml, less than 10 mg/ml, less than 5 mg/ml, less than 3 mg/ml, or less than 1.5 mg/ml,

3) wherein the starter culture comprises at least one lactose-deficient strain, which is capable of metabolizing a non-lactose carbohydrate, and

4) adding a low pH stable lactase to the process either at the start, during or at the end of the fermentation step,

wherein the low pH stable lactase retains its activity at a pH of 5.0 and a temperature of 37 °C at a level of at least 5 % as compared to its activity at the optimum pH of the lactase.

Lactose-deficient lactic acid bacteria typically grow on a non-lactose carbohydrate source, such as sucrose, galactose and glucose, added to the milk in an amount measured so as stop the fermentation process and the growth of the lactic acid bacteria by depletion of the added carbohydrate source. Hereby, the post-acidification during subsequent storage is lowered significantly or even fully prevented. A low pH stable lactase will be active during the full course of a fermentation process and hence allows conversion of most or all of the lactose present in milk to glucose and galactose. Hereby, it is possible to produce a fermented milk product with a reduced content of lactose or a lactose-free product. Also, it is possible to produce a fermented milk product with an increased natural sweetness, as glucose and galactose has a much higher sweetness than lactose.

The present invention is based on the recognition that by using a combination of a low pH stable lactase and lactose-deficient lactic acid bacteria, it is possible to obtain a fermented milk product, which at the same time has reduced lactose content, an increased sweetness and reduced post-acidification. Furthermore, it has surprisingly been found that the said combination results in a fermented milk product having an increased texture as compared to using lactose-deficient lactic acid bacteria and no lactase and as compared to using a low pH stable lactase and lactose-positive lactic acid bacteria.

Furthermore, when the lactase is added before the fermentation step the process of the invention has provided a possibility of reducing or even eliminating the amount of non-lactose carbohydrate to be added to the milk. Thus, the glucose and galactose formed by the enzymatic action of lactase will be available for the growth of the lactose-deficient lactic acid bacteria.

Finally, when the lactase is added after the fermentation step, it is possible to take full advantage of the ability of the lactose-deficient lactic acid bacteria to avoid post-acidification.

Worldwide, a significant numbers of consumers are intolerant or sensitive to lactose. Therefore, there is presently a high demand for dairy products, including fermented milk products, with a reduced content of lactose or which is substantially free of lactose. The present invention has provided a new approach for producing such product in a simple and cost-efficient manner.

DETAILED DISCLOSURE OF THE INVENTION

## Lactase

The lactase of the fermented milk product of the invention may be any lactase, which retains its activity at a pH of 5.0 and a temperature of 37 °C at a level of at least 5 % as compared to its activity at the optimum pH of the lactase.

In relation to the present invention the activity in LAU of the lactase is measured as specified in the "Definitions" section below.

In a preferred embodiment of the invention, the lactase retains its activity at a pH of 5.0 and a temperature of 37 °C at a level of at least 10 %, preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In a preferred embodiment of the invention, the lactase retains its activity at a pH of 4.0 and a temperature of 37 °C at a level of at least 5 %, preferably at least 10 %, more preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In a preferred embodiment of the invention, the lactase retains its activity at a pH of 3.0 and a temperature of 37 °C at a level of at least 5 %, preferably at least 10 %, more preferably at least 20 %, more preferably at least 30 %, more preferably at least 40 %, more preferably at least 50 %, more preferably at least 60 %, more preferably at least 70 %, and most preferably at least 80 %, as compared to its activity at the optimum pH of the lactase.

In connection with the present invention the optimum pH of the lactase is determined by measuring the lactase activity at pH using the method indicated in the "Definitions" section below and determining the pH with optimum activity. In a preferred embodiment of the invention, the lactase retains its activity at a temperature of 10 °C and a pH of 6.0 at a level of at least 10 % as compared to its activity at the optimum temperature of the lactase. Preferably, the lactase retains its activity at a temperature of 10 °C and a pH of 6.0 at a level of at least 20 %, more preferably at least 30 %, more preferably at least 40 %, more preferably at least 50 %, more preferably at least 60 %, more preferably at least 70 %, and most preferably at least 80 %, as compared to its activity at the optimum temperature of the lactase.

In connection with the present invention the optimum temperature of the lactase is determined by measuring the lactase activity at different temperatures using the method indicated in the "Definitions" section below and determining the temperature with optimum activity.

In a preferred embodiment, the lactase to be used in the product of the present invention has a lactase activity at 37°C and pH 5 which is at least 55%, such as at least 60%, at least 65%, at least 70% or at least 75%, of its lactase activity at 37°C and pH 6.

In another preferred embodiment, the lactase to be used in the product of the present invention has a lactase activity at 37°C and pH 4.5 which is at least 10%, such as at least 20%, at least 30%, at least 35% or at least 40%, of its lactase activity at 37°C and pH 6.

In another preferred embodiment, the lactase to be used in the product of the present invention has a pH optimum of the lactase activity at 37°C which is above pH 5.5.

In another preferred embodiment, the lactase to be used in the product of the present invention has a lactase activity at a temperature of 52°C and a pH of 6.5 which is at least 50%, such as at least 55%, at least 60%, at least 65%, at least 70%, at least 75% or at least 80%, of its lactase activity at a temperature of 38°C and a pH of 6.5.

In a preferred embodiment of the present invention, Km of the lactase at 5°C is below 25 mM, such as below 20 mM, below 15 mM or below 10 mM. In another preferred embodiment, Km of the lactase at 37°C is below 25 mM, such as below 20 mM or below 15 mM. The skilled person will know how to determine Km for the lactase activity at a specific temperature. Km may be determined by the method described in WO2009/071539.

In another preferred embodiment, the enzyme when hydrolyzing the lactose in the milk product has a ratio of lactase to transgalactosylase activity of more than 1:1, such as more than 2:1 or more than 3:1. In another preferred embodiment, the enzyme treatment is performed under conditions where the lactase activity of the enzyme is higher than the transgalactosylase activity, such as at least two times higher or at least three times higher.

The ratio of lactase to transgalactosylase activity in the milk product may, e.g., be determined by HPLC analysis. In another preferred embodiment, the enzyme treatment is performed under conditions where at least 50% (w/w%) of the hydrolyzed lactose is converted into free galactose. In another preferred embodiment, the enzyme treatment is performed under conditions where the hydrolyzed lactose is converted into equal amounts of free glucose and free galactose.

**EP 3 821 712 B2**

A lactase in the context of the present invention is a glycoside hydrolase having the ability to hydrolyze the disaccharide lactose into constituent galactose and glucose monomers. The group of lactases, to which the lactase of the invention belongs, comprises but is not limited to enzymes assigned to subclass EC 3.2.1.108. Enzymes assigned to other subclasses, such as, e.g., EC 3.2.1.23, may also be lactases in the context of the present invention. A lactase in the context of the invention may have other activities than the lactose hydrolyzing activity, such as for example a transgalactosylating activity. In the context of the invention, the lactose hydrolyzing activity of the lactase may be referred to as its lactase activity or its beta-galactosidase activity.

Enzymes having lactase activity to be used in a method of the present invention may be of animal, of plant or of microbial origin. Preferred lactases are obtained from microbial sources, in particular from a filamentous fungus or yeast, or from a bacterium.

The enzyme may, e.g., be derived from a strain of *Agaricus,* e.g. *A. bisporus; Ascovaginospora; Aspergillus,* e.g. *A. niger, A. awamori, A. foetidus, A. japonicus, A. oryzae; Candida; Chaetomium; Chaetotomastia; Dictyostelium,* e.g. *D. discoideum; Kluveromyces,* e.g. *K. fragilis, K. lactis; Mucor,* e.g. *M. javanicus, M. mucedo, M. subtilissimus; Neurospora,* e.g. *N. crassa; Rhizomucor,* e.g. *R. pusillus; Rhizopus,* e.g. *R. arrhizus, R. japonicus, R. stolonifer; Sclerotinia,* e.g. *S. libertiana; Torula; Torulopsis; Trichophyton,* e.g. *T. rubrum; Whetzelinia,* e.g. *W. sclerotiorum; Bacillus,* e.g. *B. coagulans, B. circulans, B. megaterium, B. novalis, B. subtilis, B. pumilus, B. stearothermophilus, B. thuringiensis; Bifidobacterium,* e.g. *B. longum, B. bifidum, B. animalis; Chryseobacterium; Citrobacter,* e.g. *C. freundii; Clostridium,* e.g. *C. perfringens; Diplodia,* e.g. *D. gossypina; Enterobacter,* e.g. *E. aerogenes, E. cloacae Edwardsiella, E. tarda; Erwinia,* e.g. *E. herbicola; Escherichia,* e.g. *E. coli; Klebsiella,* e.g. *K. pneumoniae; Miriococcum; Myrothesium; Mucor; Neurospora,* e.g. *N. crassa; Proteus,* e.g. *P. vulgaris; Providencia,* e.g. *P. stuartii; Pycnoporus,* e.g. *Pycnoporus cinnabarinus, Pycnoporus sanguineus; Ruminococcus,* e.g. *R. torques; Salmonella,* e.g. *S. typhimurium; Serratia,* e.g. *S. liquefasciens, S. marcescens; Shigella,* e.g. *S. flexneri; Streptomyces,* e.g. *S. antibioticus, S. castaneoglobisporus, S. violeceoruber; Trametes; Trichoderma,* e.g. *T. reesei, T. viride; Yersinia,* e.g. *Y. enterocolitica.*

In a preferred embodiment, the lactase originates from a bacterium, e.g. from the family Bifidobacteriaceae, such as from the genus *Bifidobacterium,* such as from a strain of *B. bifidum, B. animalis* or *B. longum.* In a more preferred embodiment, the lactase originates from *Bifidobacterium bifidum.*

In a preferred embodiment, an enzyme having lactase activity to be used in the product of the present invention comprises an amino acid sequence which is at least 50% identical to a sequence selected from the group consisting of amino acids 28-1931 of SEQ ID NO: 1, amino acids 28-1331 of SEQ ID NO: 2, SEQ ID NO: 3, SEQ ID NO: 4 and lactase active fragments thereof. In a more preferred embodiment, the enzyme comprises an amino acid sequence which is at least 60%, such as at least 70%, at least 80%, at least 90%, at least 95% or at least 98% identical to a sequence selected from the group consisting of amino acids 28-1931 of SEQ ID NO: 1, amino acids 28-1331 of SEQ ID NO: 2, SEQ ID NO: 3, SEQ ID NO: 4 and lactase active fragments thereof.

A preferred enzyme is a lactase having a sequence which is at least 50%, such as at least 60%, at least 70%, at least 80%, at least 90%, at least 95% or at least 98% identical to amino acids 28-1931 of SEQ ID NO: 1 or to a lactase active fragment thereof. Such lactase active fragment of SEQ ID NO: 1 may be any fragment of SEQ ID NO: 1 having lactase activity. A lactase active fragment of SEQ ID NO: 1 may be, e.g., amino acids 28-979, amino acids 28-1170, amino acids 28-1323, amino acids 28-1331, or amino acids 28-1600 of SEQ ID NO: 1.

In a preferred embodiment, an enzyme having lactase activity to be used in the product of the present invention comprises an amino acid sequence which is at least 50% identical to amino acids 28-1331 of SEQ ID NO: 2. In a more preferred embodiment, the enzyme comprises an amino acid sequence which is at least 60%, such as at least 70%, at least 80%, at least 90%, at least 95% or at least 98% identical to amino acids 28-1331 of SEQ ID NO: 2.

In another embodiment, an enzyme having lactase activity to be used in product of the present invention has an amino acid sequence which is at least 50% identical to SEQ ID NO: 3. Preferably, the enzyme has an amino acid sequence which is at least 60%, such as at least 70%, at least 80%, at least 90%, at least 95% or at least 98% identical to SEQ ID NO: 3.

In another embodiment, an enzyme having lactase activity to be used in the product of the present invention has an amino acid sequence which is at least 50% identical to SEQ ID NO: 4. Preferably, the enzyme has an amino acid sequence which is at least 60%, such as at least 70%, at least 80%, at least 90%, at least 95% or at least 98% identical to SEQ ID NO: 4.

For purposes of the present invention, the degree of identity between two amino acid sequences is determined using the Needleman-Wunsch algorithm (Needleman and Wunsch (1970) J. Mol. Biol. 48: 443-453) as implemented in the Needle program of the EMBOSS package (EMBOSS: The European Molecular Biology Open Software Suite, Rice et al. (2000) Trends in Genetics 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labelled "longest identity" (obtained using the -no brief option) is used as the percent identity and is calculated as follows:

5

(Identical Residues x 100)/(Length of Alignment - Total Number of Gaps in Alignment)

A particular commercial lactase suitable for use in the present invention is Lactase F "Amano" 100SD available from Amano Enzyme Europe.

Lactases to be used in a method of the present invention may be extracellular. They may have a signal sequence at their N-terminus, which is cleaved off during secretion.

Lactases to be used in a method of the present invention may be derived from any of the sources mentioned herein. The term "derived" means in this context that the enzyme may have been isolated from an organism where it is present natively, i.e. the identity of the amino acid sequence of the enzyme are identical to a native enzyme. The term "derived" also means that the enzymes may have been produced recombinantly in a host organism, the recombinantly produced enzyme having either an identity identical to a native enzyme or having a modified amino acid sequence, e.g. having one or more amino acids which are deleted, inserted and/or substituted, i.e. a recombinantly produced enzyme which is a mutant and/or a fragment of a native amino acid sequence. Within the meaning of a native enzyme are included natural variants. Furthermore, the term "derived" includes enzymes produced synthetically by, e.g., peptide synthesis. The term "derived" also encompasses enzymes which have been modified e.g. by glycosylation, phosphorylation etc., whether in vivo or in vitro. With respect to recombinantly produced enzyme the term "derived from" refers to the identity of the enzyme and not the identity of the host organism in which it is produced recombinantly.

The lactase may be obtained from a microorganism by use of any suitable technique. For instance, a lactase enzyme preparation may be obtained by fermentation of a suitable microorganism and subsequent isolation of a lactase preparation from the resulting fermented broth or microorganism by methods known in the art. The lactase may also be obtained by use of recombinant DNA techniques. Such method normally comprises cultivation of a host cell transformed with a recombinant DNA vector comprising a DNA sequence encoding the lactase in question and the DNA sequence being operationally linked with an appropriate expression signal such that it is capable of expressing the lactase in a culture medium under conditions permitting the expression of the enzyme and recovering the enzyme from the culture. The DNA sequence may also be incorporated into the genome of the host cell. The DNA sequence may be of genomic, cDNA or synthetic origin or any combinations of these, and may be isolated or synthesized in accordance with methods known in the art.

Lactases to be used in a method of the present invention may be purified. The term "purified" as used herein covers lactase enzyme protein essentially free from insoluble components from the production organism. The term "purified" also covers lactase enzyme protein essentially free from insoluble components from the native organism from which it is obtained. Preferably, it is also separated from some of the soluble components of the organism and culture medium from which it is derived. More preferably, it is separated by one or more of the unit operations: filtration, precipitation, or chromatography.

Accordingly, the enzyme having lactase activity may be purified, viz. only minor amounts of other proteins being present. The expression "other proteins" relate in particular to other enzymes. The term "purified" as used herein also refers to removal of other components, particularly other proteins and most particularly other enzymes present in the cell of origin of the lactase. The lactase may be "substantially pure", i.e. free from other components from the organism in which it is produced, i.e., e.g., a host organism for recombinantly produced lactase. Preferably, the lactase is an at least 40% (w/w) pure enzyme protein preparation, more preferably at least 50%, 60%, 70%, 80% or even at least 90% pure.

The term enzyme having lactase activity includes whatever auxiliary compounds that may be necessary for the enzyme's catalytic activity, such as, e.g., an appropriate acceptor or cofactor, which may or may not be naturally present in the reaction system.

The enzyme may be in any form suited for the use in question, such as, e.g., in the form of a dry powder or granulate, a non-dusting granulate, a liquid, a stabilized liquid, or a protected enzyme.

**Lactose-deficient lactic acid bacteria**

The terms "deficiency in lactose metabolism" and "lactose deficient" are used in the context of the present invention to characterize LAB which either partially or completely lost the ability to use lactose as a source for cell growth or maintaining cell viability. Respective LAB are capable of metabolizing one or several carbohydrates selected from sucrose, galactose and/or glucose or another fermentable carbohydrate. Since these carbohydrates are not naturally present in milk in sufficient amounts to support fermentation by lactose deficient mutants, it is necessary to add these carbohydrates to the milk. Lactose deficient and partially deficient LAB can be characterized as white colonies on a medium containing lactose and X-Gal.

In a particular embodiment of the invention, the lactose-deficient strain is capable of metabolizing a non-lactose carbohydrate selected from the group consisting of sucrose, galactose and glucose, preferably sucrose. In a particular embodiment of the invention, the lactose-deficient strain is capable of metabolizing galactose.

In a particular embodiment of the invention, the lactose-deficient strain is selected from the group consisting of:

(a) a *Streptococcus thermophilus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952;
(ii) or a strain derived from DSM 28952, wherein the derived strain is further characterized as having the ability to generate white colonies on a medium containing lactose and X-Gal;

(b) a *Streptococcus thermophilus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28953;
(ii) or a strain derived from DSM 28953, wherein the derived strain is further characterized as having the ability to generate white colonies on a medium containing lactose and X-Gal;

(c) a *Lactobacillus delbrueckii* ssp. *bulgaricus* strain, which strain is:

(i) the strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910;
(ii) or a strain derived from DSM 28910, wherein the derived strain is further characterized as having the ability to generate white colonies on a medium containing lactose and X-Gal.

In a particular embodiment of the invention, the starter culture contains both at least one *Streptococcus thermophilus* and at least one *Lactobacillus delbrueckii* subsp. *bulgaricus,* and wherein all *Streptococcus thermophilus* and all *Lactobacillus delbrueckii* subsp. *bulgaricus* strains are lactose-deficient.

## Steps of the process of the invention

In a particular embodiment of the invention, the fermentation step is terminated by a method selected from the group consisting of 1) acidification of the fermented milk rendering at least one strain of the starter culture unable to grow, 2) cooling treatment and 3) depletion of the non-lactose carbohydrate.

In a particular embodiment of the invention, non-lactose carbohydrate is added to the milk base at the start of the fermentation step.

Preferably, the non-lactose carbohydrate is added to the milk base in an amount measured so as to become depleted and hence result in stopping the growth of lactic acid bacteria and in stopping the fermentation. Preferably, the non-lactose carbohydrate is added to the milk base in an amount measured so as to become depleted and hence result in stopping the growth of lactic acid bacteria and in stopping the fermentation at a selected target pH value.

The amount of non-lactose carbohydrate to be added to the milk base depends on a number of parameters, including the lactic acid bacteria strains used in the starter culture, the composition of the milk base, the fermentation temperature and the desired target pH. Also, the amount of non-lactose carbohydrate to be added to the milk base may depend on the type and amount of lactase used in the process. The amount of non-lactose carbohydrate to be added to the milk base can be determined by experimentation, and it is well within the skills of a skilled person to carry out such experimentation.

In a particular embodiment of the invention, the added non-lactose carbohydrate is selected from the group consisting of sucrose, galactose and glucose, preferably sucrose.

In a first aspect of the invention, the low pH stable lactase is added to the milk base at the start of the fermentation step. In this aspect the added lactase will convert the lactose of the milk base to glucose and galactose, which will be available for metabolization for the starter culture in addition to the added non-lactose carbohydrate. In this case, it will not be possible to stop the fermentation by depletion of the added non-lactose carbohydrate. Thus, in a particular embodiment of the first aspect of the invention, the fermentation step is terminated by a method selected from the group consisting of 1) acidification of the fermented milk rendering at least one strain of the starter culture unable to grow, and 2) cooling treatment.

In a particular embodiment of the first aspect of the invention no non-lactose carbohydrate is added to the fermentation step and at least one lactose-deficient lactic acid strain of the starter culture is capable of metabolizing a carbohydrate selected from the group consisting of glucose and galactose. In this embodiment the lactose-deficient lactic acid strain of the starter culture grows solely on the glucose and/or galactose formed by the enzymatic action of the low pH lactase. In this embodiment of the invention, the fermentation is stopped at a target pH value by a method selected from the group consisting of 1) acidification of the fermented milk rendering at least one strain of the starter culture unable to grow, 2) cooling treatment and 3) depletion of the glucose and/or galactose formed by the low pH stable lactase.

In a second aspect of the invention, the low pH stable lactase is added to the fermented milk at the end of the fermentation. In this case, the lactase-containing fermented milk product is preferably stored at a temperature of at least 2 °C for at least 1 day. In a particular embodiment of the process of the invention, the lactase-containing fermented milk product is stored for at least two days, preferably at least 3 days, more preferably at least 4 days, more preferably at least 5 days, more preferably at least 6 days, and most preferably at least 7 days.

In a particular embodiment of the second aspect of the invention, the fermentation step is terminated by depletion of the non-lactose carbohydrate.

In a particular embodiment of the invention the target pH is between 3.2 and 4.8, more preferably between 3.6 and 4.6, more preferably between 3.8 and 4.5 and most preferably between 4.0 and 4.4.

In a particular embodiment of the invention the fermentation temperature is between 35 °C and 45 °C, preferably between 37 °C and 43 °C, and more preferably between 40 °C and 43 °C.

In another particular embodiment of the invention the fermentation is between 15 °C and 35 °C, preferably between 25 °C and 35 °C, and more preferably between 30 °C and 33 °C.

In a particular embodiment of the invention, the fermented milk product is packaged at a temperature between 15 and 45 °C.

In a particular embodiment of the invention, the pH value of the fermented milk product is maintained within a range of 0.3 pH units, preferably within a range of 0.2 pH units and most preferably within a range of 0.1 pH units, when stored after termination of the fermentation at the temperature used for fermentation over a period of 20 hours.

In a particular embodiment of the invention, the amount of added non-lactose carbohydrate is from 1 mg/g to 30 mg/g, preferably from 2 mg/g to 20 mg/g, and more preferably from 3 mg/g to 10 mg/g milk base.

In a particular embodiment of the invention, the amount of added non-lactose carbohydrate is from 0.1 % to 10 %, preferably from 0.2 % to 8 %, preferably from 0.3 % to 2 %, preferably from 0.4 % to 1.5 %, and more preferably from 0.5 % to 1.2 %, wherein % is (w/w) based on milk base.

The starter culture may have the strain composition of any conventional starter culture of lactic acid bacteria, including single strain culture and culture blends, used for producing a specific type of fermented milk product. Other useful bacteria, which may be added to the product in addition to the starter culture, include the probiotic bacteria *Bifidobacterium spp.*

In a particular embodiment of the invention, the fermented milk product after fermentation is subjected to a heat treatment so as to reduce the level of bacteria of the starter culture to no more than 1X10exp02 CFU per g fermented milk product. In this case a particular embodiment is characterized in that the lactase is added after the heat treatment. In this case the lactase-containing fermented milk product is stored at a temperature of at least 2 °C for at least 1 day. In a particular embodiment of the process of the invention, the lactase-containing fermented milk product is stored for at least two days, preferably at least 3 days, more preferably at least 4 days, more preferably at least 5 days, more preferably at least 6 days, and most preferably at least 7 days.

The heat treatment so as to reduce the level of bacteria of the starter culture to no more than 1.0X10exp02 CFU per g fermented milk is preferably carried out by subjecting the starter culture fermented milk product to a temperature of between 50 °C and 110 °C, preferably between 50 °C and 100 °C, preferably between 50 °C and 90 °C, preferably between 60 °C and 85 °C, more preferably between 65 °C and 82 °C, and most preferably between 70 °C and 80 °C. The heat treatment is preferably carried out for a period of between 5 seconds and 180 seconds, preferably between 5 seconds and 120 seconds, more preferably between 5 seconds and 90 seconds, more preferably between 5 seconds and 60 seconds, more preferably between 8 seconds and 50 seconds and most preferably between 10 and 40 seconds. Preferably, the level of bacteria of the starter culture is reduced to no more than 1.0X10exp01 CFU per g fermented milk, more preferably 0 CFU per g. Fermented milk products subjected to a heat treatment so as to reduce the level of bacteria to no more than 1X10exp02 CFU per g are suitable for use storage at ambient temperature, such as storage at a temperature of at least 5 °C, preferably at least 10 °C, more preferably at least 15 °C, and most preferably at least 20 °C.

The low pH stable lactase is added in a suitable amount to achieve the desired degree of lactose hydrolysis under the chosen reaction conditions. In a particular embodiment of the invention, lactase is added in an amount of between 100 and 20000 LAU per liter milk base, preferably between 100 and 10000 LAU per liter milk base, preferably between 100 and 5000 LAU per liter milk base, preferably less than 3000, such as less than 1500, less than 1000, less than 750 or less than 500, LAU per liter milk base.

In a preferred embodiment, the lactase is added at a concentration of between 5 and 400 LAU per g lactose in the milk base, preferably between 5 and 200 LAU per g lactose in the milk base, preferably between 5 and 100 LAU per g lactose in the milk base, preferably less than 50, such as less than 40, less than 30, less than 20 or less than 10, LAU per g lactose in the milk base.

In a preferred embodiment of the invention, the lactase is added to the milk base in an amount of between 2.0 mg/ml and 50 mg/ml, preferably between 5 mg/ml and 48 mg/ml, more preferably between 10 mg/ml and 46 mg/ml, and most preferably between 20 mg/ml and 45 mg/ml.

In a preferred embodiment of the invention, the residual level of lactose at the end of fermentation is less than 20 mg/ml, more preferably less than 15 mg/ml, more preferably less than 10 mg/ml, more preferably less than 5 mg/ml, more preferably less than 3 mg/ml, and most preferably less than 1.5 mg/ml.

In a preferred embodiment of the invention, the milk base at the start of the fermentation step has a content of lactose of between 30.0 mg/ml and 70 mg/ml, preferably between 35 mg/ml and 65 mg/ml, more preferably between 40 mg/ml and 60 mg/ml, and most preferably between 50 mg/ml and 60 mg/ml.

In a preferred embodiment of the invention, wherein the low pH-stable lactase is added to the process at the end of the fermentation step, the fermented milk product, to which the lactase is to be added, has a viscosity, which allows easy distribution of the lactase in the fermented milk product, e.g. by mixing.

In a preferred embodiment of the process of the invention, the lactase to be added to the process is provided in a sterile formulation. In another preferred embodiment of the process of the invention the lactase is added to the process under aseptic conditions, e.g. by sterile filtration of a solution of the lactase.

## Fermented milk product

The present invention relates to a fermented milk product according to the claims.

The present invention relates to a fermented milk product produced by the process disclosed above comprising the starter culture of step 3) of the said process and the low pH stable lactase added in step 4) of the said process.

In a particular embodiment of the invention, the fermented milk product is selected form the group consisting of yogurt, cream cheese, sour milk, sour cream, buttermilk, fermented whey, cultured milk, Smetana, Kefir, drinking yogurt, and Yakult. Preferably, the yogurt is selected from the group consisting of set yogurt, stirred yogurt and drinking yogurt.

In a preferred embodiment of the invention, the fermented milk product contains a further food product selected from the group consisting of fruit beverage, fermented cereal products, chemically acidified cereal products, soy milk products and any mixture thereof.

The fermented milk product typically contains protein in a level of between 2.0 % by weight to 3.5 % by weight. The fermented milk product may also be a low protein product with a protein level of between 1.0 % by weight and 2.0 % by weight. Alternatively, the fermented milk product may be a high protein product with a protein level of above 3.5 % by weight.

## Use

Also disclosed but not part of the present invention is a use in a process for producing a fermented milk product comprising the steps of

1) adding a starter culture comprising at least one lactic acid bacteria strain to a milk base, and
2) fermenting the milk for a period of time until a target pH is reached, of
3) the starter culture comprising at least one lactose-deficient strain, which is capable of metabolizing a non-lactose carbohydrate, and
4) a low pH stable lactase added to the process either at the start, during or at the end of the fermentation step, wherein the low pH stable lactase retains its activity at a pH of 5.0 and a temperature of 37 °C at a level of at least 5 % as compared to its activity at the optimum pH of the lactase.

## Definitions

In connection with the present invention the following definitions apply:

"LAU" means "Lactose Units" and 1 lactase unit (1 LAU) is the amount of enzyme which releases 1 micromole glucose per minute in M-buffer at pH 6.5 and 37°C with a lactose concentration of 4.75% w/v. M-buffer is prepared by dissolving 3.98 g $C_6H_5Na_3O_7$-$2H_2O$, 8.31 g citric acid, 0.9 g $K_2SO_4$, 2.6 g $K_2HPO_4$, 7.35 g $KH_2PO_4$, 5.45 g KOH, 4.15 g $MgCl_2$-$6H_2O$, 3.75 g $CaCl_2$-$2H_2O$ and 1.4 g $NaHCO_3$ in 4 liter water, adding 12.5 ml 4N NaOH, adjusting to pH 6.5 using HCl, and adding water up to a total volume of 5 liter.

The activity in LAU of a specific lactase may be determined by direct measurement of glucose released from lactose under the conditions described above. The skilled person will know how to determine such activity. Alternatively, the activity may be determined by using the lactase activity assay described in Example 1 of the present application. Here, the activity is obtained by comparing to a standard curve run with a lactase of known activity, and the activity of the unknown sample calculated from this. The lactase of known activity may, e.g., be Lactozym obtained from Novozymes A/S, Denmark.

The expression "heat treatment" means any treatment using any temperature, for any period of time and by any means or equipment, which inactivates at least a portion of the bacteria of the starter culture. In this connection the term "inactivate" means any stop, reduction or inhibition of growth of the bacteria, e.g. cell lysing. The expression "lactic acid bacteria" ("LAB") designates a gram-positive, microaerophilic or anaerobic bacteria, which ferment sugars with the production of acids including lactic acid as the predominantly produced acid, acetic acid and propionic acid. The industrially most useful lactic acid bacteria are found within the order "Lactobacillales" which includes *Lactococcus spp., Streptococcus spp., Lactobacillus spp., Leuconostoc spp.,* Pseudoleuconostoc spp., *Pediococcus spp., Brevibacterium spp., Enterococcus spp.* and *Propionibacterium spp.* These are frequently used as food cultures alone or in combination with other lactic acid bacteria.

Lactic acid bacteria, including bacteria of the species Lactobacillus sp. and Lactococcus sp., are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product or a cheese. Such lactic acid bacterial cultures are in general referred to as "starter cultures" or "starters". Typically, a starter culture for yogurt comprises *Streptococcus thermophilus* and *Lactobacillus delbrueckii* subsp. *bulgaricus,* and in most countries a yogurt is by legislation defined as a fermented milk product produced using a starter culture comprising the two said strains.

The term "milk" is to be understood as the lacteal secretion obtained by milking of any mammal, such as cows, sheep, goats, buffaloes or camels. In a preferred embodiment, the milk is cow's milk. The term milk also includes protein/fat solutions made of plant materials, e.g. soy milk.

The term "milk base" may be any raw and/or processed milk material that can be subjected to fermentation according to the method of the invention. Thus, useful milk bases include, but are not limited to, solutions/-suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk base may originate from any mammal, e.g. being substantially pure mammalian milk, or reconstituted milk powder.

Prior to fermentation, the milk base may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk base to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of dairy products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a dairy product in solid (such as a cheese) or liquid form (such as a fermented milk product).

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The expression "fermented milk product" means a food or feed product wherein the preparation of the food or feed product involves fermentation of a milk base with a lactic acid bacteria. "Fermented milk product" as used herein includes but is not limited to products such as thermophilic fermented milk products, e.g. yoghurt, mesophilic fermented milk products, e.g. sour cream and buttermilk, as well as fermented whey.

The term "thermophile" herein refers to microorganisms that thrive best at temperatures above 35°C. The industrially most useful thermophilic bacteria include Streptococcus spp. and Lactobacillus spp. The term "thermophilic fermentation" herein refers to fermentation at a temperature above about 35°C, such as between about 35°C to about 45°C. The term "thermophilic fermented milk product" refers to fermented milk products prepared by thermophilic fermentation of a thermophilic starter culture and include such fermented milk products as set-yoghurt, stirred-yoghurt and drinking yoghurt, e.g. Yakult.

The term "mesophile" herein refers to microorganisms that thrive best at moderate temperatures (15°C-35°C). The industrially most useful mesophilic bacteria include Lactococcus spp. and Leuconostoc spp. The term "mesophilic fermentation" herein refers to fermentation at a temperature between about 22°C and about 35°C. The term "mesophilic fermented milk product" refers to fermented milk products prepared by mesophilic fermentation of a mesophilic starter culture and include such fermented milk products as buttermilk, sour milk, cultured milk, smetana, sour cream, Kefir and fresh cheese, such as quark, tvarog and cream cheese.

In connection with the present invention, "shear stress" may be measured by the following method: Seven days after production, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows: Holding time (to rebuild to somewhat original structure)

5 minutes without any physical stress (oscillation or rotation) applied to the sample. Oscillation step (to measure the elastic and viscous modulus, G' and G", respectively, therefore calculating the complex modulus G*)

Constant strain = 0.3 %, frequency (f) = [0.5...8] Hz
6 measuring points over 60 s (one every 10 s)

Rotation step (to measure shear stress at 300 1/s)

Two steps were designed:
1) Shear rate = [0.3-300] 1/s and 2) Shear rate = [275-0.3] 1/s.

Each step contained 21 measuring points over 210 s (on every 10 s).

The shear stress at 300 1/s was chosen for further analysis, as this correlates to mouth thickness when swallowing a fermented milk product.

In connection with the present invention, "gel firmness" may be measured by the following method: Gel firmness is measured by a back extrusion test with a texture analyzer (TA.XT Plus, Stable Micro System, Surrey, UK) supplied with a 35mm parallel plate. The travel distance is set to 15 mm, and the travel speed to 2 mm/s. The test is performed after 7 days from production. The fermented milk product was brought to 13°C and manually stirred gently, and measured in a 250 g plastic container. The maximal force (N or g) obtained by force versus distance curves is used as "gel firmness" parameter, the positive area (N* mm) as degree of deformation, the maximal negative force (N) as ropiness.

The term "low pH stable lactase" herein refers to a lactase, which retains its activity at a pH of 5.0 and a temperature of 37 °C at a level of at least 5 % as compared to its activity at the optimum pH of the lactase.

The term "activity at the optimum pH" means the lactase activity at the pH, where the lactase has its optimum activity.

The term "non-lactose carbohydrate" means any carbohydrate, which is not lactose, and which a lactose-deficient lactic acid bacterium used in the process of the invention is capable of metabolizing.

The expression "at the start of the fermentation step" means shortly before, at the same time as or shortly after addition of the starter culture to the milk base. Here, the term "shortly" means less than 30 minutes".

The expression "during the fermentation step" means at any time during the fermentation after the start and before the end of the fermentation.

The expression "at the end of the fermentation step" means shortly before, at the same time as or shortly after the target pH is reached. Here, the term "shortly" means less than 30 minutes".

The term "target pH" means the pH at which the fermentation step ends. Depending on various parameters of the process, the fermentation step is terminated by a method selected from the group consisting of 1) acidification of the fermented milk rendering at least one strain of the starter culture unable to grow, 2) cooling treatment and 3) depletion of the non-lactose carbohydrate.

## DEPOSITS AND EXPERT SOLUTION

The Applicant requests that a sample of the deposited microorganism should be made available only to an expert approved by the Applicant.

*Streptococcus thermophilus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28952.

Streptococcus thermophilus strain deposited with DSMZ-Deutsche Sammlung von Mikroorganismen und Zel-Ikulturen GmbH, Inhoffenstr. 7B, D-38124 Braunschweig, on 2014-06-12 under the accession no. DSM 28953.

*Lactobacillus delbrueckii* ssp. *bulgaricus* strain deposited with DSMZ-Deutsche Sammlung von Mikroorganismen und Zellkulturen GmbH, Inhoffenstr. 7B, D-38124 Braunschweig, on 2014-06-12 under the accession no. DSM 28910;

The deposits were made according to the Budapest treaty on the international recognition of the deposit of microorganisms for the purposes of patent procedure.

## EXAMPLES

## Example 1

*Lactase activity-assay in Eppendorf tubes at 37$\underline{o}$C, pH 6.5*

Principle:

Lactase hydrolyses lactose into glucose and galactose. Glucose is measured after a modified version of the common glucose oxidase / peroxidase assay (Werner, W. et al. (1970) Z. analyt. Chem. 252: 224.).

LAU is defined as the amount of enzyme liberating 1 micromole of glucose per min at 37°C, pH 6.5 in M-buffer (M-buffer is defined in the description of the present patent application). Alternatively, the activity in LAU for a specific lactase may be determined by the method described here. The value obtained is compared to a standard curve run with a lactase of known activity, and the activity of the unknown sample calculated from this. The lactase of known activity may, e.g., be Lactozym obtained from Novozymes A/S, Denmark.

Solutions:

Assay buffer: 50 mM succinate, 50 mM HEPES, 50 mM CHES, 150 mM KCl, 2 mM CaCl$_2$, 1 mM MgCl$_2$, 0.01% Triton X100, pH 6.5

GOD-Perid solution: 65 mM sodium phosphate, pH 7, 0.4 g/l Glucose oxidase, 0.013 g/l HRP (Horse Radish Peroxidase), 0.65 g/l ABTS (2,2'-azino-bis(3-ethylbenzthiazoline-6-sulphonic acid)).

Substrate:

160 mM lactose, 50 mM succinate, 50 mM HEPES, 50 mM CHES, 150 mM KCl, 2 mM CaCl$_2$, 1 mom MgCl$_2$, pH 6.5.

Standard:

Lactozym (available from Novozymes A/S, Denmark) with a known activity in LAU/g is used as standard, diluted in assay buffer in the range from 0.09 - 0.7 LAU/g.

Samples:

Enzyme samples are diluted appropriately in assay buffer.

Procedure:

1. 375 $\mu$l substrate is incubated 5 minutes at 37°C.
2. 25 $\mu$l enzyme diluted in assay buffer is added.
3. The reaction is stopped after 30 minutes by adding 60 $\mu$l 1 M NaOH
4. 20 $\mu$l is transferred to a 96 well microtiter plate and 200 $\mu$l GOD-Perid solution is added. After 30 minutes at room temperature, the absorbance is measured at 420 nm.

## Example 2

100 ml 15 or 30%(w/w) whey permeate containing primarily lactose and ions was made by mixing 15 or 30 g spray-dried whey permeate powder (Variolac, Aria) in 85 or 70 ml ionic water respectively. The solution was poured in a flask containing a magnetic stirring bar and placed in a water bath at 37°C. After 15 min, enzyme was added. Enzymes tested were Lactozym, a commercially available lactase from Novozymes A/S, Denmark, having an activity of 3060 LAU/g, and an experimental lactase from *Bifidobacterium bifidum* having the encoded sequence shown in SEQ ID NO: 2 and an activity of 295 LAU/g.

Dosages were 4225 LAU/l milk of Lactozym and 2025 LAU/l milk of the *Bifidobacterium* lactase. Milk samples were taken at regular intervals up till 5.5 hrs. and the enzyme was inactivated by heating to 99°C for 10 min in a thermomixer. Samples were diluted appropriately and filtered through a 0.20 $\mu$m filter.

Lactose hydrolysis was measured using a Dionex BioLC equipped with a Dionex PA1 column and a Pulsed Amperiometrisk Detektor (PAD). Peaks were identified and quantified by comparing with known standards of lactose, glucose and galactose.

Results are given below.

Table 1: Lactose, glucose and galactose in 15% DS whey permeate after treatment with Lactozym or *Bifidobacterium* lactase.

| | Lactozym | | | *Bifidobacterium* lactase | | |
|---|---|---|---|---|---|---|
| Time min | Lactose mM | Glucose mM | Galactose mM | Lactose mM | Glucose mM | Galactose mM |
| 0 | 499 | 1 | 2 | 499 | 1 | 2 |
| 30 | 312 | 135 | 106 | 410 | 61 | 63 |
| 60 | 211 | 224 | 155 | 349 | 119 | 122 |
| 120 | 110 | 295 | 221 | 220 | 199 | 202 |
| 180 | 66 | 324 | 249 | 149 | 281 | 290 |
| 240 | 50 | 346 | 279 | 84 | 336 | 348 |
| 330 | 37 | 372 | 312 | 31 | 350 | 368 |

Table 2: Lactose, glucose and galactose in 30% DS whey permeate after treatment with Lactozym or *Bifidobacterium* lactase.

| | Lactozym | | | *Bifidobacterium* lactase | | |
|---|---|---|---|---|---|---|
| Time min | Lactose mM | Glucose mM | Galactose mM | Lactose mM | Glucose mM | Galactose mM |
| 0 | 848 | 1 | 4 | 848 | 1 | 4 |
| 30 | 824 | 109 | 75 | 819 | 43 | 45 |
| 60 | 615 | 253 | 150 | 788 | 86 | 83 |
| 120 | 420 | 370 | 242 | 651 | 159 | 158 |
| 180 | 291 | 459 | 300 | 625 | 232 | 230 |
| 240 | 246 | 559 | 373 | 501 | 283 | 273 |
| 330 | 154 | 544 | 367 | 391 | 333 | 324 |
| 1440 | 54 | 649 | 545 | 20 | 727 | 739 |

Also when tested at higher lactose concentrations as in 15% or 30% whey permeate no or very little galactooligosaccharides are produced. Again, the produced galactose and glucose levels are equal and match the amount of lactose hydrolyzed. For comparison, Lactozym produces less galactose than glucose, clearly showing that galactooligosaccharides have been produced.

## Example 3

*pH profile (at 37°C) and temperature profile (at pH 6.5) of experimental lactase from Bifidobacterium bifidum using lactose as substrate.*

Principle:

Lactase hydrolyses lactose and glucose + galactose is formed. Glucose is measured after a modified version of the common glucose oxidase / peroxidase assay (Werner, W. et al. (1970) Z. analyt. Chem. 252: 224.)

*pH profile*

Substrate:

167 mM lactose, 50 mM succinate, 50 mM HEPES, 50 mM CHES, 150 mM KCl, 2 mM $CaCl_2$, 1 mM $MgCl_2$ and pH adjusted to pH 3, 4, 5, 6, 7, 8, 9 and 10 with NaOH.

Enzyme Sample:

Experimental lactase from *Bifidobacterium bifidum* having the encoded sequence shown in SEQ ID NO: 2 was diluted appropriately in 150 mM KCl, 2 mM $CaCl_2$, 1 mM $MgCl_2$, 0.01% Triton X100.

Procedure:

- 10 $\mu$l enzyme sample diluted in enzyme dilution buffer was added to PCR tubes at room temp.
- 90 $\mu$l substrate was added at room temp. and quickly placed in a Peltier Thermal Cycler (PCT-200, MJ research) at 37°C and incubated for 30 min and then placed on ice.
- The reaction was stopped by adding 100 $\mu$l 0.25 M NaOH.
- 20 $\mu$l was transferred to a 96 well microtitre plate and 230 $\mu$l 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Table 3:

| pH | *B. bifidum* lactase relative activity (% of activity at pH6) |
|---|---|
| 3 | 0 |
| 4 | 4 |
| 5 | 75 |
| 6 | 100 |
| 7 | 85 |
| 8 | 39 |
| 9 | 10 |
| 10 | 4 |

*Temperature profile*

Substrate:

167 mM lactose, 50 mM succinate, 50 mM HEPES, 50 mM CHES, 150 mM KCl, 2 mM $CaCl_2$, 1 mM $MgCl_2$ and pH adjusted to pH 6.5 with NaOH.

Enzyme Sample:

Experimental lactase from *Bifidobacterium bifidum* having the encoded sequence shown in SEQ ID NO: 2 was diluted appropriately in 50 mM succinate, 50 mM HEPES, 50 mM CHES, 150 mM KCl, 2 mM $CaCl_2$, 1 mM $MgCl_2$, 0.01% Triton X100 and pH adjusted to pH 6.5.

Procedure:

- 10 µl enzyme sample diluted in enzyme dilution buffer was added to PCR tubes at room temp.
- 90 µl preheated (in a Peltier Thermal Cycler 30-70°C) substrate was added and incubation was performed with a temp. gradient from 30-70°C for 30 min. and then placed on ice.
- The reaction was stopped by adding 100 µl 0.25 M NaOH.
- 20 µl was transferred to a 96 well microtitre plate and 230 µl 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Table 4:

| Temp. °C | B. bifidum lactase relative activity (% of activity at 38.1 °C) |
|---|---|
| 20 | 54 |
| 21 | 63 |
| 22 | 64 |
| 24 | 68 |
| 26 | 73 |
| 29 | 81 |
| 31 | 88 |
| 34 | 94 |
| 36 | 96 |
| 38 | 100 |
| 43 | 96 |
| 48 | 91 |
| 52 | 83 |
| 57 | 76 |
| 62 | 58 |
| 66 | 32 |
| 69 | 20 |
| 70 | 17 |

## Example 4

*Determination of Km for lactase enzymes at 5°C*

Principle:

Lactase hydrolyses lactose and glucose + galactose is formed. Glucose is measured after a modified version of the common glucose oxidase / peroxidase assay (Werner, W. et al. (1970) Z. analyt. Chem. 252: 224.)

Substrate:

Different lactose concentrations ranging from Km/5 to 10* Km, 50 mM succinate, 50 mM HEPES, 50 mM CHES, 150 mM KCl, 2 mM $CaCl_2$, 1 mM $MgCl_2$ and pH adjusted to pH 6.5 with NaOH.

Enzyme Sample:

Experimental lactase from *Bifidobacterium bifidum* having the encoded sequence shown in SEQ ID NO: 2 was diluted appropriately in 50 mM succinate, 50 mM HEPES, 50 mM CHES, 150 mM KCl, 2 mM $CaCl_2$, 1 mM $MgCl_2$, 0.01% Triton X100, pH adjusted to pH 6.5 with NaOH.
12 g/l Lactozym (commercially available lactase from Novozymes A/S, Denmark) was diluted 6000 times in the same buffer.

Procedure:

- 10 µl enzyme sample (5°C) was added to a 96 well microtitre plate on ice.
- 90 µl substrate (5°C) was added and incubated for 2 hours at 5°C.
- The reaction was stopped by adding 100 µl 0.25 M NaOH.
- 20 µl was transferred to a 96 well microtitre plate and 230 µl 65 mM sodium phosphate, pH 7, 0.4 g/l Glucose oxidase, 0.013 g/l HRP, 0.65 g/l ABTS solution was added. After 30 minutes at room temperature, the absorbance was measured at 420 nm.

Km determination:

Computerized nonlinear least-squares fitting and the Michaelis-Menten equation:

$$v = (Vmax * S)/(Km + S)$$

was used. Km for the *Bifidobacterium* lactase and Lactozym were determined to be 8 mM and 30 mM, respectively.
In a similar test performed at 37°C, Km for the *Bifidobacterium* lactase and Lactozym were determined to be 13 mM and 30 mM, respectively.

## Example 5

*Production of yogurt using lactase from Bifidobacterium bifidum and a lactose-deficient starter culture - Different levels of sucrose and lactase*

Experimental plan

For the selected combination of a low pH stable lactase and a lactose-deficient starter culture, two levels of added sucrose (0.5 % and 0.7 %), and two levels of added lactase (800 LAU/L and 1000 LAU/L), were tested. As reference fermentations with no added lactase and two levels of sucrose (1.0 % and 1.5 %) were carried out. The lactase was added at the start of the fermentation together with the starter culture.

Milk base

Table 5: Composition of milk base

|  | Amount (g) | Protein content (%) | Carbohydrate (%) | Fat (%) |
|---|---|---|---|---|
| Skim milk | 1000 | 3.5 | 4.8 | 0.1 |
| 1.5 % milk | 2000 | 3.4 | 4.7 | 1.5 |
| Protein powder "Promilk 802FB" | 14 | 80.0 |  |  |
| Protein powder "Milex 240" | 95 | 34.0 |  |  |
| Total milk base | 3109 | 4.71 | 4.57 | 1.00 |

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

Fat, protein and lactose levels were determined using MilkoScan analysis. Post-acidification was measured over a period of 28 days storage at 5 °C.

The level of sucrose, glucose, galactose, fructose and lactose in the fermented milk at day 1 and day 14 after production was measured by HPLC.

*Gel firmness*

Gel firmness is measured by a back extrusion test with a texture analyzer (TA.XT Plus, Stable Micro System, Surrey, UK) supplied with a 35mm parallel plate. The travel distance is set to 15 mm, and the travel speed to 2 mm/s. The test is performed after 7 days from production. The fermented milk product was brought to 13°C and manually stirred gently, and measured in a 250 g plastic container. The maximal force (g) obtained by force versus distance curves is used as "gel firmness" parameter.

*Shear stress*

Seven days after incubation, the fermented milk product was brought to 13°C and manually stirred gently by means of a spoon (5 times) until homogeneity of the sample. The rheological properties of the sample were assessed on a rheometer (Anton Paar Physica Rheometer with ASC, Automatic Sample Changer, Anton Paar® GmbH, Austria) by using a bob-cup. The rheometer was set to a constant temperature of 13 °C during the time of measurement. Settings were as follows:
Holding time (to rebuild to somewhat original structure)
5 minutes without any physical stress (oscillation or rotation) applied to the sample. Oscillation step (to measure the elastic and viscous modulus, G' and G", respectively, therefore calculating the complex modulus G*)

Constant strain = 0.3 %, frequency (f) = [0.5...8] Hz
6 measuring points over 60 s (one every 10 s)

Rotation step (to measure shear stress at 300 1/s)

Two steps were designed:
1) Shear rate = [0.3-300] 1/s and 2) Shear rate = [275-0.3] 1/s.
Each step contained 21 measuring points over 210 s (on every 10 s).
The shear stress at 300 1/s was chosen for further analysis, as this correlates to mouth thickness when swallowing a fermented milk product.

Procedure

The ingredients of the milk base were mixed and allowed to re-hydrate for 2 hours at 5 °C. The milk base was then pasteurized at 90 °C for 20 minutes. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 °C.

Results

*Post-acidification*

Table 6: Post-acidification

|  | Ref. 1 | Ref. 2 | Test Sample 1 | Test Sample 2 | Test Sample 3 | Test Sample 4 |
|---|---|---|---|---|---|---|
| Sucrose (%) | 1 | 1.5 | 0.5 | 0.5 | 0.7 | 0.7 |
| Lactase (LAU/L) | 0 | 0 | 800 | 1000 | 800 | 1000 |
| Ferment. time (Hours: Min.) | 7:25 | 7:05 | 7:15 | 7:15 | 7:00 | 7:00 |
| End pH | 4.58 | 4.55 | 4.55 | 4.55 | 4.55 | 4.55 |
| pH Day 1 | 4.58 | 4.53 | 4.54 | 4.53 | 4.50 | 4.51 |
| pH Day 7 | 4.54 | 4.49 | 4.50 | 4.50 | 4.47 | 4.49 |
| pH Day 14 | 4.49 | 4.43 | 4.49 | 4.49 | 4.46 | 4.47 |
| pH Day 21 | 4.35 | 4.34 | 4.45 | 4.45 | 4.42 | 4.43 |
| pH Day 28 | 4.35 | 4.34 | 4.45 | 4.42 | 4.43 | 4.42 |
| pH drop | 0.23 | 0.19 | 0.09 | 0.11 | 0.07 | 0.09 |

For all six samples, i.e. 2 reference samples and 4 samples produced according to the invention (test samples), a pH of about 4.55 was reached in 7 hours.

As will appear from Table 6, the post-acidification over a period of 28 days was strongly reduced for the samples produced according to the invention as compared to the reference samples.

*Carbohydrate analysis*

Table 7: Residual carbohydrate levels after 1 day from production

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase (LAU/L) | 0 | 0 | 800 | 1000 | 800 | 1000 |
| Fructose (mg/g) | 1.62 | 1.91 | 0.80* | 0.74* | 1.27 | 1.18 |
| Galactose (mg/g) | < 0.9 | 1.78* | 29.01 | 29.87 | 28.78 | 29.29 |
| Glucose (mg/g) | 0.77* | 1.73 | 27.60 | 27.84 | 28.08 | 28.19 |
| Lactose (mg/g) | 54.16 | 53.37 | 1.18 | 0.40 | 1.20 | 0.40 |
| Sucrose (mg/g) | < 0.3 | 4.6 | < 0.3 | < 0.3 | 1.3 | 0.7 |
| * value is between Limit of Detection and Limit of Quantification. | | | | | | |

All levels of Table 7 are mean values of two samples.

For the 4 test samples, at the end of fermentation the level of lactose was very low and the level of glucose and galactose was very high as compared to the reference samples indicating high activity of the added lactase. For the test samples using a lactase level of 1000 LAU/L a residual lactose level of approx. 0.04 % was obtained, and for the test samples using a lactase level of 800 LAU/L a residual lactose level of approx. 0.1 % was obtained.

Fermented milk having a low lactose level and a high level of glucose and galactose has a much higher level of perceived sweetness than fermented milk with a high lactose level and a low level of glucose and galactose like the reference samples. The reason for this is that glucose and galactose has a much higher sweetness index than lactose.

*Gel firmness*

Table 8: Gel firmness

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase (LAU/L) | 0 | 0 | 800 | 1000 | 800 | 1000 |
| Gel firmness (g) | 45.76 | 47.42 | 48.19 | 54.57 | 50.19 | 46.60 |

All levels of Table 8 are mean values of two samples.

As will appear from Table 8, 3 out of the 4 test samples had significantly higher gel firmness than the 2 reference samples.

*Shear stress*

Table 9: Shear stress measured at 300 s$^{-1}$

|  | Ref. 1 | Ref. 2 | Test Sample 1 | Test Sample 2 | Test Sample 3 | Test Sample 4 |
|---|---|---|---|---|---|---|
| Sucrose (%) start ferment. | 1 | 1.5 | 0.5 | 0.5 | 0.7 | 0.7 |
| Lactase (LAU/L) | 0 | 0 | 800 | 1000 | 800 | 1000 |
| Shear stress (Pa) | 67.1 | 65.3 | 78.2 | 84.4 | 84.7 | 77.6 |

All levels of Table 9 are mean values of two samples.

As will appear from Table 9, the 4 test samples had significantly higher shear stress measured at 300 s$^{-1}$ than the 2 reference samples. In the group of 4 test samples, the combinations of 1) a level of added sucrose of 0.5 % and a lactase level of 1000 LAU/L, and 2) a level of added sucrose of 0.7 % and a lactase level of 800 LAU/L, had the highest shear stress.

## Example 6

*Production of yogurt using lactase from Bifidobacterium bifidum and a lactose-deficient starter culture - addition of excess level of sucrose before fermentation I*

Experimental plan

Fermentations were carried out with a reference sample containing no lactase and 9.5 % sucrose and 2 test samples containing 800 LAU/L of lactase and 6.5 % and 7.0 % sucrose. The lactase was added at the start of the fermentation together with the starter culture.

Milk base

Table 10: Composition of basic milk base 1

|  | Amount (g) | Protein content (%) | Carbohydrate (%) | Fat (%) |
|---|---|---|---|---|
| Skim milk | 984 | 3.5 | 4.8 | 0.1 |
| 1.5 % milk | 1911 | 3.4 | 4.7 | 1.5 |
| Protein powder "Promilk 802FB" | 13.3 | 80.0 |  |  |
| Protein powder "Milex 240" | 91.5 | 34.0 | 54.00 | 1.00 |
| Total milk base | 3000 | 4.71 | 4.57 | 1.02 |

Table 11: Composition of basic milk base 2

|  | Amount (g) | Protein content (%) | Carbohydrate (%) | Fat (%) |
|---|---|---|---|---|
| Skim milk | 984 | 3.5 | 4.8 | 0.1 |
| 1.5 % milk | 1911 | 3.4 | 4.7 | 1.5 |
| Protein powder "Promilk 802FB" | 17.40 | 80.0 |  |  |
| Protein powder "Milex 240" | 109.50 | 34.0 | 5400 | 1.00 |
| Sucrose | 180.00 |  | 100.0 |  |
| Total milk base | 3202 | 4.70 | 11.75 | 0.96 |

Basic milk base 2 was used to prepare the final milk bases for the reference sample and the two test samples by addition of additional sucrose.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

All measurements were carried out using the same methods as in Example 5.

Procedure

The ingredients of the milk base were mixed and allowed to re-hydrate for 2 hours at 5 °C. The milk base was then pasteurized at 95 °C for 5 minutes. The milk base was then homogenized at 65 °C at 200 bar. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 $^0$C.

Results

*Post-acidification*

Table 12: Post-acidification

| Sample | 1 | 2 | 3 |
|---|---|---|---|
| Lactase (LAU/L) | 0 | 800 | 800 |
| Fermentation time | 6 h 15 min | 6 h 15 min | 6 h 15 min |
| End pH | 4.55 | 4.51 | 4.51 |
| pH Day 4 | 4.54 | 4.44 | 4.44 |
| pH Day 7 | 4.51 | 4.42 | 4.42 |
| pH Day 14 | 4.40 | 4.38 | 4.37 |
| pH Day 21 | 4.38 | 4.38 | 4.37 |
| pH Day 28 | 4.36 | 4.37 | 4.36 |
| pH drop | 0.19 | 0.14 | 0.15 |

*Carbohydrate analysis*

Table 13: Carbohydrate analysis

| Sample | 1 | | 2 | | 3 | |
|---|---|---|---|---|---|---|
| Lactase (LAU/L) | 0 | | 800 | | 800 | |
| Sucrose (%) start ferment. | 9.5 | | 6.5 | | 7.0 | |
| | Day 1 | Day 28 | Day 1 | Day 28 | Day 1 | Day 28 |
| Fructose (mg/g) | 2.4 | 3.07 | 1.7 | 2.01 | 1.9 | 2.00 |
| Galactose (mg/g) | < 2.0 | 8.99 | 26.4 | 29.03 | 26.4 | 28.72 |
| Glucose (mg/g) | < 1.0 | 8.84 | 26.2 | 27.52 | 26.2 | 27.22 |
| Lactose (mg/g) | 51.4 | 38.04 | 2.8 | <2 | 2.7 | <2 |
| Sucrose (mg/g | 64.3 | 64.23 | 41.7 | 42.38 | 46.0 | 46.26 |

All levels of Table 13 are mean values of two samples.

As will appear from Table 13, for the 2 test samples, at the end of fermentation the level of lactose was very low and the level of glucose and galactose was very high as compared to the reference sample indicating high activity of the added lactase. Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

*Gel firmness and shear stress*

Table 14: Gel firmness and shear stress at 300 $s^{-1}$

| Sample | 1 | | 2 | | 3 | |
|---|---|---|---|---|---|---|
| Lactase (LAU/L) | 0 | | 800 | | 800 | |
| Sucrose (%) start ferment. | 9.5 | | 6.5 | | 7.0 | |
| Gel firmness (g) | 46.36 | | 46.76 | | 45.75 | |
| Shear stress at 1 s-1 (Pa) | 9.4 | 9.2 | 9.3 | 8.8 | 10.3 | 10.2 |
| Shear stress at 30.2 s-1 (Pa) | 39.2 | 38.8 | 41.6 | 39.2 | 44.7 | 45.1 |
| Shear stress at 135 s-1 (Pa) | 58.3 | 58.0 | 67.2 | 64.5 | 72.3 | 72.2 |
| Shear stress at 300 s-1 (Pa) | 69.4 | 69.1 | 80.6 | 78.2 | 84.4 | 84.4 |

As will appear from Table 14, the shear stress at 300 $s^{-1}$ of the two test samples containing lactase was significantly increased as compared to the reference sample with no lactase.

## **Example 7**

*Production of yogurt using lactase from Bifidobacterium bifidum and a lactose-deficient starter culture - addition of excess level of sucrose before fermentation II*

Experimental plan

Fermentations were carried out with two reference samples containing no lactase and 1.5 % and 9.5 % sucrose and 3 test samples containing 1000 LAU/L of lactase and 0.5 %, 6.5 % and 7.0 % sucrose. The lactase was added at the start of the fermentation together with the starter culture.

Milk base

The two basic milk bases of Example 6 were used to prepare the final milk bases for the reference samples and the three test samples by addition of additional sucrose.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Measurements

All measurements were carried out using the same methods as in Example 5.

Procedure

The ingredients of the milk base were mixed and allowed to re-hydrate for 2 hours at 5 °C. The milk base was then pasteurized at 90 °C for 20 minutes. The fermentation was carried out at 43 °C to an end pH of 4.55 to form yogurt. The yogurt was cooled in a PTU (Post Treatment Unit) at a cooling temperature of 25 °C at 2 bars. The cooled yogurt was stored at 6 °C.

Results

*Post-acidification*

Table 15: Post-acidification

| Sample | 1 | 2 | 3 | 4 | 5 |
|---|---|---|---|---|---|
| Lactase (LAU/L) | 0 | 1000 | 0 | 1000 | 1000 |
| Fermentation time | 6 h 50 min | 6 h 55 min | 6 h 35 min | 6 h 10 min | 5 h 40 min |
| End pH | 4.55* | 4.55* | 4.55* | 4.55* | 4.55* |
| pH Day 1 | 4.54 | 4.51 | 4.52 | 4.48 | 4.47 |
| pH Day 7 | 4.45 | 4.45 | 4.40 | 4.34 | 4.35 |
| pH Day 14 | 4.37 | 4.44 | 4.37 | 4.32 | 4.34 |
| pH Day 21 | 4.35 | 4.39 | 4.35 | 4.29 | 4.29 |
| pH Day 28 | 4.34 | 4.37 | 4.33 | 4.27 | 4.26 |
| pH drop | 0.21 | 0.18 | 0.22 | 0.28 | 0.29 |
| * The fermentation is stopped by cooling at pH = 4.55. | | | | | |

As will appear from Table 15, the post-acidification was at the same level for the reference samples containing no lactase and the test samples containing lactase.

*Carbohydrate analysis*

Table 16: Carbohydrate analysis Day 1

| Sample | 1 | 2 | 3 | 4 | 5 |
|---|---|---|---|---|---|
| Lactase (LAU/L) | 0 | 1000 | 0 | 1000 | 1000 |
| Sucrose (%) start ferment. | 1.5 | 0.5 | 9.5 | 6.5 | 7.0 |
| Fructose (mg/g) | 1.96 | < 1 | 2.70 | 1.74 | 1.88 |
| Galactose (mg/g) | <2 | 29.19 | <2 | 27.83 | 27.89 |
| Glucose (mg/g) | < 1 | 27.37 | < 1 | 27.29 | 27.40 |
| Lactose (mg/g) | 56.11 | <2 | 54.56 | <2 | <2 |
| Sucrose (mg/g | 5.45 | <4 | 63.44 | 39.79 | 45.44 |

Table 17: Carbohydrate analysis Day 28

| Sample | 1 | 2 | 3 | 4 | 5 |
|---|---|---|---|---|---|
| Lactase (LAU/L) | 0 | 1000 | 0 | 1000 | 1000 |
| Sucrose (%) start ferment. | 1.5 | 0.5 | 9.5 | 6.5 | 7.0 |
| Fructose (mg/g) | 1.88 | < 1 | 4.72 | 2.95 | 3.39 |
| Galactose (mg/g) | 3.90 | 28.83 | <2 | 28.79 | 29.15 |
| Glucose (mg/g) | 2.69 | 26.78 | 1.66 | 26.83 | 27.27 |
| Lactose (mg/g) | 50.71 | <2 | 52.42 | <2 | <2 |
| Sucrose (mg/g | 4.18 | <4 | 62.07 | 39.91 | 46.17 |

As will appear from Tables 16 and 17, for the 3 test samples, at the end of fermentation the level of lactose was very low and the level of glucose and galactose was very high as compared to the reference sample indicating high activity of the added lactase.

Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

*Gel firmness and shear stress*

Table 18: Gel firmness and shear stress

| Sample | 1 | | 2 | | 3 | | 4 | | 5 | |
|---|---|---|---|---|---|---|---|---|---|---|
| Lactase (LAU/L) | 0 | | 1000 | | 0 | | 1000 | | 1000 | |
| Sucrose (%) start ferment. | 1.5 | | 0.5 | | 9.5 | | 6.5 | | 7.0 | |
| Gel firmness (g) | 51.13 | | 49.41 | | 45.04 | | 45.94 | | 47.89 | |
| Shear stress at 1 s-1 (Pa) | 9.4 | 9.3 | 8.4 | 8.5 | 8.1 | 8.5 | 7.7 | 7.8 | 8.3 | 8.5 |
| Shear stress at 30.2 s-1 (Pa) | 39.4 | 38.5 | 38.2 | 37.6 | 34.3 | 34.7 | 37.3 | 37.1 | 38.8 | 38.9 |
| Shear stress at 135 s-1 (Pa) | 61.0 | 59.5 | 65.8 | 65.1 | 56.8 | 56.8 | 72.7 | 72.3 | 74.8 | 74.9 |
| Shear stress at 300 s-1 (Pa) | 72.1 | 70.5 | 78.8 | 77.6 | 69.1 | 69.1 | 86.7 | 86.6 | 89.4 | 89.4 |

As will appear from Table 18, the shear stress of the three test samples containing lactase was significantly increased as compared to the reference sample with no lactase.

## Reference Example 8

Production of yogurt using lactase from Bifidobacterium bifidum and a lactose-deficient starter culture - addition of lactase before and after fermentation

Experimental plan

Table 19: Experimental plan

| Sample | Timing of lactase addition | Milk Base | Sucrose (%) | Lactase (LAU/L) |
|--------|---------------------------|-----------|-------------|-----------------|
| 1 | None | 1 | 0.70 | 0 |
| 2 | Together with culture | 1 | 0.70 | 600 |
| 3 | Together with culture | 1 | 0.70 | 800 |
| 4 | Together with culture | 1 | 0.70 | 1000 |
| 5 | Together with culture | 1 | 0.70 | 1400 |
| 6 | None | 2 | 0.97 | 0 |
| 7 | After fermentation | 2 | 0.97 | 800 |
| 8 | After fermentation | 2 | 0.97 | 1600 |
| 9 | After fermentation | 2 | 0.97 | 2400 |
| 10 | After fermentation | 2 | 0.97 | 3200 |

The fermentation temperature was 43 °C. The end pH was 4.50. The ingredients of the milk base were mixed and allowed to re-hydrate for 2 hours at 6 °C. The milk base was then pasteurized at 95 °C for 5 minutes and homogenized at 200/50 bar at 65 °C. The fermentation was carried out at 43 °C to an end pH of 4.50 to form yogurt. The yogurt was cooled to 6 °C. The cooled yogurt was stored at 6 °C.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Milk base

Table 20: Composition of milk base 1

| | Amount (g) | Protein content (%) | Carbohydrate (%) | Fat (%) |
|--|-----------|---------------------|------------------|---------|
| Sucrose | 22 | 0.0 | 100.0 | 0.0 |
| 0.5 % milk | 1500 | 3.8 | 4.8 | 0.5 |
| 1.5 % milk | 1500 | 3.6 | 4.7 | 1.5 |
| Aria Skimmed Milk Powder | 103 | 34.0 | 54.0 | 1.0 |
| Total milk base | 3125 | 4.67 | 7.05 | 0.98 |

Table 21: Composition of milk base 2

|  | Amount (g) | Protein content (%) | Carbohydrate (%) | Fat (%) |
|---|---|---|---|---|
| Sucrose | 30 | 0.0 | 100.0 | 0.0 |
| 0.5 % milk | 1500 | 3.8 | 4.8 | 0.5 |
| 1.5 % milk | 1500 | 3.6 | 4.7 | 1.5 |
| Aria Skimmed Milk Powder | 103 | 34.0 | 54.0 | 1.0 |
| Total milk base | 3133 | 4.66 | 7.30 | 1.00 |

Measurements

All measurements were carried out using the same methods as in Example 5.

Results

*Post-acidification*

Table 22: Post-acidification

| Sample | Timing of lactase addition | Milk Base | Lactase (LAU/L) | pH Day 0 | pH Day 7 | pH Day 28 | pH Day 42 | pH drop |
|---|---|---|---|---|---|---|---|---|
| 1 | None | 1 | 0 | 4.89* | 4.89* | 4.87 | 4.84 | 0.05 |
| 2 | Together with culture | 1 | 600 | 4.26 | 4.23 | 4.23 | 4.22 | 0.04 |
| 3 | Together with culture | 1 | 800 | 4.22 | 4.26 | 4.24 | 4.24 | -0.02 |
| 4 | Together with culture | 1 | 1000 | 4.32 | 4.26 | 4.26 | 4.26 | 0.06 |
| 5 | Together with culture | 1 | 1400 | 4.36 | 4.30 | 4.28 | 4.27 | 0.09 |
| 6 | None | 2 | 0 | 4.47 | 4.47 | 4.47 | 4.44 | 0.03 |
| 7 | After fermentation | 2 | 800 | 4.43 | 4.40 | 4,42 | 4.38 | 0.05 |
| 8 | After fermentation | 2 | 1600 | 4.44 | 4.40 | 4,42 | 4.39 | 0.05 |
| 9 | After fermentation | 2 | 2400 | 4.41 | 4.43 | 4.40 | 4.40 | 0.01 |
| 10 | After fermentation | 2 | 3200 | NA | 4.43 | 4.41 | 4.40 | NA |
| * Insufficient sucrose to obtain fermentation to target pH. Therefore, this sample cannot be used as reference. | | | | | | | | |

As will appear from Table 22, the post-acidification over a period of 42 days was at a low level of below 0.09 for all lactase levels tested with lactase addition at the start of fermentation and at an even lower level of below 0.05 for all lactase levels tested with lactase addition at the end of fermentation. The addition of lactase at the end of the fermentation did not result in a statistically different post-acidification as compared to the reference sample with no added lactase. Thus, the present experiment shows that the use of a low pH stable lactase in combination with a lactose-deficient starter culture does not result in an unacceptable increase of the low post-acidification, which may be obtained with such a starter culture. This is true both when the lactase is added at the start and at the end of fermentation.

*Gel firmness and shear stress*

Table 23: Gel firmness and Shear Stress

| Sample | Timing of lactase addition | Milk Base | Sucrose (%) | Lactase (LAU/L) | Gel firmness (g) | Shear Stress (Pa) |
|---|---|---|---|---|---|---|
| 1 | None | 1 | 0.70 | 0 | 0.187* | 46.4* |
| 2 | Together with culture | 1 | 0.70 | 600 | 0.281 | 63.9 |
| 3 | Together with culture | 1 | 0.70 | 800 | 0.302 | 65.3 |
| 4 | Together with culture | 1 | 0.70 | 1000 | 0.318 | 64.5 |
| 5 | Together with culture | 1 | 0.70 | 1400 | 0.307 | 63.7 |
| 6 | None | 2 | 0.97 | 0 | 0.304 | 53.1 |
| 7 | After fermentation | 2 | 0.97 | 800 | 0.312 | 51.4 |
| 8 | After fermentation | 2 | 0.97 | 1600 | 0.322 | 52.8 |
| 9 | After fermentation | 2 | 0.97 | 2400 | 0.323 | 52.9 |
| 10 | After fermentation | 2 | 0.97 | 3200 | 0.320 | 51.7 |
| * Insufficient sucrose to obtain fermentation to target pH. Therefore, this sample cannot be used as reference. | | | | | | |

As will appear from Table 23, high levels of both gel firmness and shear stress was obtained for all lactase levels tested with lactase addition at the start of fermentation. For lactase levels tested with lactase addition at the end of fermentation, the levels of gel firmness and shear stress are somewhat lower, which is due to the mixing of the lactase into the yogurt, which partly disrupts the texture of the yogurt. For all lactase levels tested with lactase addition at the end of fermentation, the gel firmness is slightly higher than the reference sample with no added lactase. For all lactase levels tested with lactase addition at the end of fermentation, the shear stress is maintained at the same level or slightly lower.

*Carbohydrate analysis*

Table 24: Carbohydrate analysis

| Sam-pie | Lactase (LAU/L) | Galactose Day 20 (mg/g) | Glucose Day 20 (mg/g) | Sucrose Day 20 (mg/g) | Lactose Day 20 (mg/g) | Lactose 24 hours (%) | Lactose Day 20 (%) |
|---|---|---|---|---|---|---|---|
| 1 | 0 | 2.4 | 2.3 | < 1.0 | 50.7 | ND | ND |
| 2 | 600 | 29.5 | 27.7 | < 1.0 | < 0.5 | > 0.1 | 0.023 |
| 3 | 800 | 30.2 | 28.1 | < 1.0 | < 0.5 | 0.028 | 0.016 |
| 4 | 1000 | 30.0 | 27.8 | < 1.0 | < 0.5 | 0.014 | 0.014 |
| 5 | 1400 | 29.5 | 27.4 | < 1.0 | < 0.5 | 0.006 | 0.013 |
| 6 | 0 | 4.2 | 4.2 | < 1.0 | 49.2 | ND | ND |
| 7 | 800 | 31.2 | 30.7 | < 1.0 | < 0.5 | > 0.2 | 0.014 |
| 8 | 1600 | 30.7 | 30.2 | < 1.0 | < 0.5 | > 0.1 | 0.012 |
| 9 | 2400 | 31.2 | 30.6 | < 1.0 | < 0.5 | 0.029 | 0.011 |
| 10 | 3200 | 31.8 | 31.2 | < 1.0 | < 0.5 | 0.015 | 0.011 |

As will appear from Table 24, at Day 20 high concentrations of galactose and glucose are formed for all lactase levels tested both with lactase addition at the start and at the end of fermentation. Also, at Day 20 the level of glucose was below the detection limit of 0.5 mg/g, which qualifies as lactose free in some countries. For the higher levels of lactase, most of the lactose removal has been obtained 24 hours after the end of fermentation.

**Example 9**

*Production of yogurt using lactase from Bifidobacterium bifidum and a lactose-deficient starter culture - addition of lactase before fermentation II*

Experimental plan

Table 25: Experimental plan

| Sample | Sucrose (%) | Lactase (LAU/L) |
|--------|-------------|-----------------|
| 1 | 0 | 0 |
| 2 | 0 | 1000 |
| 3 | 0 | 1200 |
| 4 | 0 | 1600 |
| 5 | 0.2 | 0 |
| 6 | 0.2 | 1000 |
| 7 | 0.2 | 1200 |
| 8 | 0.2 | 1600 |
| 9 | 0.7 | 0 |
| 10 | 0.7 | 1000 |
| 11 | 0.7 | 1200 |
| 12 | 0.7 | 1600 |

The fermentation temperature was 43 °C. The end pH was 4.45. The ingredients of the milk base were mixed and allowed to re-hydrate for 2 hours at 6 °C. The milk base was then pasteurized at 95 °C for 5 minutes and homogenized at 200/50 bar at 65 °C. The fermentation was carried out at 43 °C to an end pH of 4.45 to form yogurt. The yogurt was cooled to 5 °C. The cooled yogurt was stored at 6 °C.

Starter culture

The starter culture is composed of *Streptococcus thermophilus* strain deposited under the accession no. DSM 28952, *Streptococcus thermophilus* strain deposited under the accession no. DSM 28953, and *Lactobacillus delbrueckii* subsp. *bulgaricus* strain deposited under accession no. DSM 28910.

Lactase

Lactase from *Bifidobacterium bifidum* having the encoded sequence of SEQ ID NO. 2.

Milk base

Table 26: Composition of milk base

|  | Amount (g) | Protein content (%) | Carbohydrate (%) | Fat (%) |
|--|-----------|---------------------|------------------|---------|
| 0.5 % milk | 1500 | 3.8 | 4.8 | 0.5 |
| 1.5 % milk | 1500 | 3.6 | 4.7 | 1.5 |
| Aria Skimmed Milk Powder | 103 | 34.0 | 54.0 | 1.0 |
| Total milk base | 3103 | 4.70 | 6.39 | 0.99 |

Measurements

All measurements were carried out using the same methods as in Example 5.

Results

*Post-acidification*

Table 27: Post-acidification

| Sample | Sucrose (%) | Lactase (LAU/L) | pH Day 0 | pH Day 1 | pH Day 14 | pH Day 28 | pH Day 42 | pH drop |
|---|---|---|---|---|---|---|---|---|
| 1 | 0 | 0 | ND | ND | ND | ND | ND | ND |
| 2 | 0 | 1000 | 4.45 | 4.39 | 4.36 | 4.33 | 4.39 | 0.06 |
| 3 | 0 | 1200 | 4.45 | 4.39 | 4.38 | 4.34 | 4.39 | 0.06 |
| 4 | 0 | 1600 | 4.46 | 4.39 | 4.38 | 4.34 | 4.39 | 0.07 |
| 5 | 0.2 | 0 | ND | ND | ND | ND | ND | ND |
| 6 | 0.2 | 1000 | 4.50 | 4.44 | 4.40 | 4.40 | 4.43 | 0.07 |
| 7 | 0.2 | 1200 | 4.53 | 4.47 | 4.46 | 4.42 | 4.46 | 0.07 |
| 8 | 0.2 | 1600 | 4.53 | 4.49 | 4.46 | 4.44 | 4.47 | 0.06 |
| 9 | 0.7 | 0 | 4.76 | 4.73 | 4.69 | 4.68 | 4.70 | 0.06 |
| 10 | 0.7 | 1000 | 4.44 | 4.40 | 4.39 | 4.33 | 4.38 | 0.06 |
| 11 | 0.7 | 1200 | 4.46 | 4.40 | 4.39 | 4.33 | 4.39 | 0.07 |
| 12 | 0.7 | 1600 | 4.47 | 4.41 | 4.40 | 4.34 | 4.41 | 0.06 |

As will appear from Table 27, the post-acidification over a period of 42 days was at a low very level of approx. 0.06 for all lactase and sucrose levels tested. Thus, the present experiment shows that the use of a low pH stable lactase in combination with a lactose-deficient starter culture does not result in an unacceptable increase of the low post-acidification, which may be obtained with such a starter culture.

*Gel firmness and shear stress*

Table 28: Gel firmness and Shear Stress

| Sample | Sucrose (%) | Lactase (LAU/L) | Gel firmness (g) | Shear Stress (Pa) |
|---|---|---|---|---|
| 1 | 0 | 0 | ND | ND |
| 2 | 0 | 1000 | 0.386 | 70.3 |
| 3 | 0 | 1200 | 0.377 | 69.8 |
| 4 | 0 | 1600 | 0.388 | 69.5 |
| 5 | 0.20 | 0 | ND | ND |
| 6 | 0.20 | 1000 | 0.380 | 70.7 |
| 7 | 0.20 | 1200 | 0.378 | 69.5 |
| 8 | 0.20 | 1600 | 0.397 | 70.2 |
| 9 | 0.70 | 0 | ND | ND |
| 10 | 0.70 | 1000 | 0.362 | 71.2 |
| 11 | 0.70 | 1200 | 0.375 | 72.2 |

(continued)

| Sample | Sucrose (%) | Lactase (LAU/L) | Gel firmness (g) | Shear Stress (Pa) |
|---|---|---|---|---|
| 12 | 0.70 | 1600 | 0.362 | 71.9 |

As will appear from Table 28, high levels of both gel firmness and shear stress was obtained for all lactase levels tested. The gel firmness and shear strength are at the same order of magnitude for all three levels of lactase tested.

*Carbohydrate analysis*

Table 29: Carbohydrate analysis

| Sam -pie | Sucrose (%) | Lactase (LAU/L) | Fructose (mg/g) | Galactose (mg/g) | Glucose (mg/g) | Lactose (mg/g) | Sucrose (mg/g) |
|---|---|---|---|---|---|---|---|
| 1 | 0 | 0 | < 0.5 | 6.3 | < 0.5 | 45.5 | <2 |
| 2 | 0 | 1000 | < 0.5 | 30.7 | 26.0 | < 0.9 | <2 |
| 3 | 0 | 1200 | < 0.5 | 31.5 | 26.6 | < 0.9 | <2 |
| 4 | 0 | 1600 | < 0.5 | 30.9 | 25.8 | < 0.9 | <2 |
| 5 | 0.20 | 0 | < 0.5 | 11.7 | 6.7 | 36.1 | <2 |
| 6 | 0.20 | 1000 | < 0.5 | 30.8 | 27.8 | < 0.9 | <2 |
| 7 | 0.20 | 1200 | < 0.5 | 31.6 | 28.3 | < 0.9 | <2 |
| 8 | 0.20 | 1600 | < 0.5 | 31.5 | 28.0 | < 0.9 | <2 |
| 9 | 0.70 | 0 | 1.1 | 9.1 | 9.1 | 43.4 | <2 |
| 10 | 0.70 | 1000 | 1,1 | 31.1 | 29.5 | < 0.9 | <2 |
| 11 | 0.70 | 1200 | 1.0 | 32.1 | 30.3 | < 0.9 | 1.8 |
| 12 | 0.70 | 1600 | 0.9 | 31.8 | 29.9 | < 0.9 | 1.9 |

As will appear from Table 29, for the test samples containing lactase, at the end of fermentation the level of lactose was very low and the level of glucose and galactose was very high as compared to the reference sample indicating high activity of the added lactase.

Fermented milk having a low lactose level and a high level of glucose and galactose has a much higher level of sweetness than fermented milk with a high lactose level and a low level of glucose and galactose like the reference samples. The reason for this is that glucose and galactose has a much higher sweetness than lactose.

**Sequence listing**

SEQ ID NO.: 1 shows the sequence of a mutant of SEQ ID NO. 4.
SEQ ID NO.: 2 shows the sequence of a mutant of SEQ ID NO. 4.
SEQ ID NO.: 3 shows the sequence of a lactase from *Bifidobacterium bifidum* DSM20215.
SEQ ID NO.: 4 shows the sequence of a lactase from *Bifidobacterium bifidum* NCIMB41171, the nucleotide sequence of which is listed in NCBI with the accession number DQ448279.
SEQ ID NO: 4 is discussed in the following references, wherein it is referred to as bbgIII:

Appl Microbiol Biotechnol (2007) 76:1365-1372, T K Goulas et al.
Appl Microbiol Biotechnol (2009) 82:1079-1088, T Goulas et al.
Appl Microbiol Biotechnol (2009) 84:899-907, T Goulas et al.

**Claims**

1. A fermented milk product comprising the starter culture of step 3) and the low pH stable lactase added in step 4) produced by the process comprising the steps of:

   1) adding a starter culture comprising both at least one lactose-deficient *Streptococcus thermophilus* and at least one lactose-deficient *Lactobacillus delbrueckii* subsp. *bulgaricus* to a milk base,
   2) fermenting the milk for a period of time until a target pH is reached, wherein the residual level of lactose at the end of fermentation is less than 25 mg/ml, less than 20 mg/ml, less than 15 mg/ml, less than 10 mg/ml, less than 5 mg/ml, less than 3 mg/ml, or less than 1.5 mg/ml,
   3) wherein the starter culture comprises at least one lactose-deficient strain, which is capable of metabolizing a non-lactose carbohydrate, and
   4) adding a low pH stable lactase to the process either at the start, during or at the end of the fermentation step,

   wherein the low pH stable lactase retains its activity at a pH of 5.0 and a temperature of 37°C at a level of at least 5% as compared to its activity at the optimum pH of the lactase.

2. The fermented milk product according to claim 1, wherein the low pH stable lactase retains its activity at a temperature of 10°C and a pH of 6.0 at a level of at least 10% as compared to its activity at the optimum temperature of the lactase.

3. The fermented milk product according to claim 1 or 2, the wherein lactose-deficient strain is capable of metabolizing a non-lactose carbohydrate selected from the group consisting of sucrose, galactose and glucose.

4. The fermented milk product according to any of the preceding claims, wherein non-lactose carbohydrate is added to the milk base at the start of the fermentation step.

5. The fermented milk product according to claim 4, wherein the non-lactose carbohydrate is added to the milk base in an amount measured so as to become depleted and hence result in stopping the growth of lactic acid bacteria and in stopping the fermentation.

6. The fermented milk product according to any of the preceding claims, wherein the low pH stable lactase is added to the milk base at the start of the fermentation step.

7. The fermented milk product according to claim 6, wherein no non-lactose carbohydrate is added to the fermentation step, and wherein at least one lactose-deficient lactic acid strain of the starter culture is capable of metabolizing a carbohydrate selected from the group consisting of glucose and galactose.

8. The fermented milk product according to any of claims 1-5, wherein the low pH stable lactase is added to the milk base at the end of the fermentation step.

9. The fermented milk product according to any of claims 1-8, wherein the lactose-deficient strain is selected from the group consisting of:

   (a) a *Streptococcus thermophilus* strain, which strain is:

      (i) the strain DSM 28952;
      (ii) or a strain derived from DSM 28952, wherein the derived strain is further characterized as having the ability to generate white colonies on a medium containing lactose and X-Gal;

   (b) a *Streptococcus thermophilus* strain, which strain is:

      (i) the strain DSM 28953;
      (ii) or a strain derived from DSM 28953, wherein the derived strain is further characterized as having the ability to generate white colonies on a medium containing lactose and X-Gal;

   (c) a *Lactobacillus delbrueckii* ssp. *bulgaricus* strain, which strain is:

      (i) the strain DSM 28910;

(ii) or a strain derived from DSM 28910, wherein the derived strain is further characterized as having the ability to generate white colonies on a medium containing lactose and X-Gal.

10. The fermented milk product according to any of claims 1-9, wherein the starter culture contains both at least one *Streptococcus thermophilus* and at least one *Lactobacillus delbrueckii* subsp. *bulgaricus,* and wherein all *Streptococcus thermophilus* and all *Lactobacillus delbrueckii* subsp. *bulgaricus* strains are lactose-deficient.

11. The fermented milk product according to any of the preceding claims, wherein the fermented milk product is selected form the group consisting of set yogurt, stirred yogurt, cream cheese, sour milk, sour cream, buttermilk, fermented whey, cultured milk, Smetana, Kefir, drinking yogurt, and Yakult.

12. The fermented milk product according to any of the preceding claims, wherein the fermented milk product contains a further food product selected from the group consisting of fruit beverage, fermented cereal products, chemically acidified cereal products, soy milk products and any mixture thereof.

13. The fermented milk product according to any of the preceding claims, wherein the fermented milk product contains protein in a level of between 2.0% by weight to 3.5% by weight, between 1.0% by weight and 2.0% by weight, or above 3.5% by weight.

**Patentansprüche**

1. Fermentiertes Milchprodukt, umfassend die Starterkultur aus Schritt 3) und die in Schritt 4) zugesetzte stabile Laktase mit niedrigem pH-Wert, hergestellt durch das Verfahren, das die folgenden Schritte umfasst:

   1) Zusetzen einer Starterkultur, umfassend sowohl mindestens einen lactosearmen *Streptococcus thermophilus* als auch mindestens einen lactosearmen *Lactobacillus delbrueckii* subsp. *bulgaricus,* zu einer Milchbasis,
   2) Fermentieren der Milch für eine Zeitspanne, bis ein Ziel-pH-Wert erreicht ist, wobei der Restgehalt an Lactose am Ende der Fermentation geringer als 25 mg/ml, geringer als 20 mg/ml, geringer als 15 mg/ml, geringer als 10 mg/ml, geringer als 5 mg/ml, geringer als 3 mg/ml oder geringer als 1,5 mg/ml ist,
   3) wobei die Starterkultur mindestens einen lactosearmen Stamm umfasst, der in der Lage ist, ein lactosefreies Kohlenhydrat zu metabolisieren, und
   4) Zusetzen einer stabilen Laktase mit niedrigem pH-Wert entweder zu Beginn, während oder am Ende des Fermentationsschritts,

   wobei die stabile Laktase mit niedrigem pH-Wert ihre Aktivität bei einem pH-Wert von 5,0 und einer Temperatur von 37 °C in einem Umfang von mindestens 5 % im Vergleich zu ihrer Aktivität bei dem optimalen pH-Wert der Laktase beibehält.

2. Fermentiertes Milchprodukt nach Anspruch 1, wobei die stabile Laktase mit niedrigem pH-Wert ihre Aktivität bei einer Temperatur von 10 °C und einem pH-Wert von 6,0 in einem Umfang von mindestens 10 % im Vergleich zu ihrer Aktivität bei der optimalen Temperatur der Laktase beibehält.

3. Fermentiertes Milchprodukt nach Anspruch 1 oder 2, wobei der lactosearme Stamm in der Lage ist, ein lactosefreies Kohlenhydrat, ausgewählt aus der Gruppe bestehend aus Saccharose, Galactose und Glucose, zu metabolisieren.

4. Fermentiertes Milchprodukt nach einem der vorstehenden Ansprüche, wobei der Milchbasis zu Beginn des Fermentationsschritts ein lactosefreies Kohlenhydrat zugesetzt wird.

5. Fermentiertes Milcherzeugnis nach Anspruch 4, wobei das lactosefreie Kohlenhydrat der Milchbasis in einer Menge zugesetzt wird, die so bemessen ist, dass sie erschöpft wird und somit dazu führt, dass das Wachstum der Milchsäurebakterien gestoppt und die Fermentation unterbrochen wird.

6. Fermentiertes Milchprodukt nach einem der vorstehenden Ansprüche, wobei die stabile Laktase mit niedrigem pH-Wert der Milchbasis zu Beginn des Fermentationsschritts zugesetzt wird.

7. Fermentiertes Milchprodukt nach Anspruch 6, wobei dem Fermentationsschritt kein lactosefreies Kohlenhydrat zugesetzt wird und wobei mindestens ein lactosearmer Milchsäurestamm der Starterkultur in der Lage ist, ein Kohlenhydrat, ausgewählt aus der Gruppe bestehend aus Glucose und Galactose, zu metabolisieren.

8. Fermentiertes Milchprodukt nach einem der Ansprüche 1 bis 5, wobei die stabile Laktase mit niedrigem pH-Wert der Milchbasis am Ende des Fermentationsschritts zugesetzt wird.

9. Fermentiertes Milchprodukt nach einem der Ansprüche 1 bis 8, wobei der lactosearme Stamm ausgewählt ist aus der Gruppe bestehend aus:

(a) einem *Streptococcus thermophilus-Stamm,* wobei es sich handelt um:

(i) den Stamm DSM 28952;
(ii) oder einen von DSM 28952 abgeleiteten Stamm, wobei der abgeleitete Stamm ferner **dadurch gekennzeichnet ist, dass** er die Fähigkeit besitzt, weiße Kolonien auf einem Lactose und X-Gal enthaltenden Medium zu bilden;

(b) einen *Streptococcus thermophilus-Stamm,* wobei es sich handelt um:

(i) den Stamm DSM 28953;
(ii) oder einen von DSM 28953 abgeleiteten Stamm, wobei der abgeleitete Stamm ferner **dadurch gekennzeichnet ist, dass** er die Fähigkeit besitzt, weiße Kolonien auf einem Lactose und X-Gal enthaltenden Medium zu bilden;

(c) einen *Lactobacillus delbrueckii* ssp. *bulgaricus-Stamm,* wobei es sich handelt um:

(i) den Stamm DSM 28910;
(ii) oder einen von DSM 28910 abgeleiteten Stamm, wobei der abgeleitete Stamm ferner **dadurch gekennzeichnet ist, dass** er die Fähigkeit besitzt, weiße Kolonien auf einem Lactose und X-Gal enthaltenden Medium zu bilden.

10. Fermentiertes Milchprodukt nach einem der Ansprüche 1 bis 9, wobei die Starterkultur sowohl mindestens einen *Streptococcus thermophilus* als auch mindestens einen *Lactobacillus delbrueckii* subsp. *bulgaricus* enthält, und wobei alle *Streptococcus thermophilus*- und alle *Lactobacillus delbrueckii* subsp. *bulgaricus-Stämme* lactosearm sind.

11. Fermentiertes Milchprodukt nach einem der vorstehenden Ansprüche, wobei das fermentierte Milchprodukt ausgewählt ist aus der Gruppe bestehend aus Joghurt, gerührtem Joghurt, Frischkäse, Sauermilch, saurer Sahne, Buttermilch, fermentierter Molke, kultivierter Milch, Smetana, Kefir, Trinkjoghurt und Yakult.

12. Fermentiertes Milchprodukt nach einem der vorstehenden Ansprüche, wobei das fermentierte Milchprodukt ein weiteres Lebensmittelprodukt enthält, ausgewählt aus der Gruppe bestehend aus Fruchtgetränken, fermentierten Getreideprodukten, chemisch gesäuerten Getreideprodukten, Sojamilchprodukten und einem Gemisch davon.

13. Fermentiertes Milchprodukt nach einem der vorstehenden Ansprüche, wobei das fermentierte Milchprodukt Protein in einer Menge zwischen 2,0 Gew.-% und 3,5 Gew.-%, zwischen 1,0 Gew.-% und 2,0 Gew.-% oder über 3,5 Gew.-% enthält.

**Revendications**

1. Produit laitier fermenté comprenant la culture de départ de l'étape 3) et la lactase à faible pH ajoutée à l'étape 4), produit par le procédé comprenant les étapes suivantes :

1) ajouter à une base de lait une culture de départ comprenant à la fois au moins un *Streptococcus thermophilus* pauvre en lactose et au moins un *Lactobacillus delbrueckii* subsp. *bulgaricus* pauvre en lactose,
2) fermenter le lait pendant un certain temps jusqu'à ce qu'un pH cible soit atteint, le niveau résiduel de lactose à la fin de la fermentation étant inférieur à 25 mg/ml, inférieur à 20 mg/ml, inférieur à 15 mg/ml, inférieur à 10 mg/ml, inférieur à 5 mg/ml, inférieur à 3 mg/ml ou inférieur à 1,5 mg/ml,
3) dans lequel la culture de départ comprend au moins une souche déficiente en lactose, pouvant métaboliser un glucide autre que le lactose, et

4) ajouter une lactase à faible pH stable au processus, soit au début, soit pendant, soit à la fin de l'étape de fermentation,

dans lequel la lactase à faible pH stable conserve son activité à un pH de 5,0 et à une température de 37°C à un niveau d'au moins 5 % par rapport à son activité au pH optimal de la lactase.

2. Produit laitier fermenté selon la revendication 1, dans lequel la lactase à faible pH stable conserve son activité à une température de 10°C et à un pH de 6,0 à un niveau d'au moins 10 % par rapport à son activité à la température optimale de la lactase.

3. Produit laitier fermenté selon la revendication 1 ou 2, la souche déficiente en lactose pouvant métaboliser un glucide autre que le lactose choisi dans le groupe constitué par le saccharose, le galactose et le glucose.

4. Produit laitier fermenté selon l'une quelconque des revendications précédentes, dans lequel un glucide autre que le lactose est ajouté à la base de lait au début de l'étape de fermentation.

5. Produit laitier fermenté selon la revendication 4, dans lequel le glucide autre que le lactose est ajouté à la base de lait en une quantité mesurée de manière à s'épuiser et donc à stopper la croissance des bactéries lactiques et à arrêter la fermentation.

6. Produit laitier fermenté selon l'une quelconque des revendications précédentes, dans lequel la lactase à faible pH est ajoutée à la base de lait au début de l'étape de fermentation.

7. Produit laitier fermenté selon la revendication 6, dans lequel aucun glucide autre que le lactose n'est ajouté à l'étape de fermentation, et dans lequel au moins une souche d'acide lactique déficiente en lactose de la culture de départ peut métaboliser un glucide choisi dans le groupe constitué par le glucose et le galactose.

8. Produit laitier fermenté selon l'une quelconque des revendications 1 à 5, dans lequel la lactase à faible pH est ajoutée à la base de lait à la fin de l'étape de fermentation.

9. Produit laitier fermenté selon l'une quelconque des revendications 1 à 8, dans lequel la souche déficiente en lactose est choisie dans le groupe constitué de :

   (a) une souche de *Streptococcus thermophilus,* cette souche étant :

   (i) la souche DSM 28952 ;
   (ii) ou une souche dérivée de DSM 28952, la souche dérivée étant en outre **caractérisée par** sa capacité à générer des colonies blanches sur un milieu contenant du lactose et du X-Gal ;

   (b) une souche de *Streptococcus thermophilus,* cette souche étant :

   (i) la souche DSM 28953 ;
   (ii) ou une souche dérivée de DSM 28953, la souche dérivée étant en outre **caractérisée par** sa capacité à générer des colonies blanches sur un milieu contenant du lactose et du X-Gal ;

   (c) une souche de *Lactobacillus delbrueckii* ssp. *bulgaricus,* cette souche étant :

   (i) la souche DSM 28910 ;
   (ii) ou une souche dérivée de DSM 28910, la souche dérivée étant en outre **caractérisée par** sa capacité à générer des colonies blanches sur un milieu contenant du lactose et du X-Gal.

10. Produit laitier fermenté selon l'une quelconque des revendications 1 à 9, dans lequel la culture de départ contient au moins un *Streptococcus thermophilus* et au moins un *Lactobacillus delbrueckii* subsp. *bulgaricus,* et dans lequel toutes les souches de *Streptococcus thermophilus* et toutes les souches de *Lactobacillus delbrueckii* subsp. *bulgaricus* sont déficientes en lactose.

11. Produit laitier fermenté selon l'une quelconque des revendications précédentes, dans lequel le produit laitier fermenté est choisi dans le groupe constitué par le yaourt ferme, le yaourt brassé, le fromage à la crème, le lait aigre, la crème aigre, le babeurre, le lactosérum fermenté, le lait de culture, le Smetana, le Kefir, le yaourt à boire et le Yakult.

12. Produit laitier fermenté selon l'une quelconque des revendications précédentes, dans lequel le produit laitier fermenté contient un autre produit alimentaire choisi dans le groupe constitué par les boissons aux fruits, les produits céréaliers fermentés, les produits céréaliers chimiquement acidifiés, les produits à base de lait de soja et tout mélange de ceux-ci.

13. Produit laitier fermenté selon l'une quelconque des revendications précédentes, dans lequel le produit laitier fermenté contient des protéines à un niveau compris entre 2,0 % en poids et 3,5 % en poids, entre 1,0 % en poids et 2,0 % en poids, ou supérieur à 3,5 % en poids.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009071539 A
- WO 2013160413 A
- EP 2957180 A1

**Non-patent literature cited in the description**

- **NEEDLEMAN** ; **WUNSCH**. *J. Mol. Biol.*, 1970, vol. 48, 443-453
- **RICE et al.** EMBOSS: The European Molecular Biology Open Software Suite. *Trends in Genetics*, 2000, vol. 16, 276-277
- **WERNER, W. et al.** *Z. analyt. Chem.*, 1970, vol. 252, 224
- NCBI, DQ448279
- **T K GOULAS**. *Appl Microbiol Biotechnol*, 2007, vol. 76, 1365-1372
- **T GOULAS**. *Appl Microbiol Biotechnol*, 2009, vol. 82, 1079-1088
- **T GOULAS**. *Appl Microbiol Biotechnol*, 2009, vol. 84, 899-907